# EUROPÄISCHE PATENTANMELDUNG

(11) **EP 4 684 834 A1**
(43) Veröffentlichungstag der Anmeldung: **28.01.2026**
(21) Anmeldenummer: 24191219.5
(22) Anmeldetag: 26.07.2024
(51) Int. Cl.: A61P 35/00, A61K 31/519, C07D 471/14

(54) **KRISTALLFORMEN ONKOLOGISCH WIRKSAMER IMIPRIDONE UND VERFAHREN ZU DEREN HERSTELLUNG**

(71) Anmelder: BRIU GmbH, 61462 Königstein (DE)
(72) Erfinder: SCHICK, Ursula, 69120 Heidelberg (DE); ROSE, Uwe-Bernd, 69120 Heidelberg (DE)
(74) Vertreter: Ullrich & Naumann PartG mbB

(57) **Zusammenfassung**

Die vorliegenden Erfindung betrifft die Dihydrochloridsalze von Imipridon-201 und Imipridon-206 in kristalliner Form deren das Röntgen-Pulverdiffraktogramme bei Verwendung von Cu K-α-Strahlung bei 25°C mindestens 3 der folgenden 2Θ (2 Theta)-Werte mit einer Fehlertoleranz von jeweils ± 0.2 aufweisen: bei 6,9; 7,7; 9,6; 12,9; 15,5; 21,2; 21,4; 22,5; 23,1; 25,3; und 26,8 (Imipridon-201) und 14,2, 21,3, 25,5, 26,0, und 27,7 (Imipridon-201) sowie Verfahren zu deren Herstellung und deren pharmazeutische Verwendung.

## Beschreibung

### GEBIET DER ERFINDUNG

Die vorliegende Erfindung betrifft neue röntgenographische Kristallformen der Dihydrochlorid-Salze von 7-Benzyl-4-(2-methylbenzyl)-1,2,6,7,8,9-hexahydroimidazo[1,2-a]pyrido[3,4-e]pyrimidin-5(4H)-on (Imipridon-201) und 7-Benzyl-4-(2,4-difluorobenzyl)-2,4,6,7,8,9-hexahydroimidazo[1,2-a]pyrido[3,4-e]pyrimidin-5(1H)-on (Imipridon-206) sowie Verfahren zur Herstellung dieser Kristallformen.

### HINTERGRUND DER ERFINDUNG

Imidazolinimipridone sind eine seit langem bekannte Klasse von Verbindungen, die sowohl Imidazolin- als auch Imid- oder Imidazolidin-Strukturen enthalten und die verschiedene Anwendungen haben können, hauptsächlich in der Chemie und Medizin. Beispielsweise offenbart DE 2150062 (1971 eingereicht und 1973 veröffentlicht) Imidazo-[1,2-a]-pyrido-[4,3-d]-pyrimidine, ihre Säureadditionssalze und Verfahren zu ihrer Herstellung.

Imipridon-201 ist ein erstes kleines Molekül der Klasse der Imidazolinimipridone, das selektiv an den G-Protein-gekoppelten Dopaminrezeptor D2 (DRD2) und die mitochondriale Protease ClpP bindet.

Imipridon-201 hat die Summenformel C24H26N4O, die CAS-Nummer 1616632-77-9 und den IUPAC-Namen 7-Benzyl-4-(2-methylbenzyl)-1,2,6,7,8,9-hexahydroimidazo[1,2-a]pyrido-[3,4-e]pyrimidin-5(4H)-on und wird in der Regel als ONC201 (bzw. gewinkeltes ONC201) bezeichnet. Oral verabreicht, scheint Imipridon-201 bei einigen Patienten mit bestimmten Formen von fortgeschrittenem Krebs, mit Hirntumoren, die die H3 K27M-Mutation enthalten, gut verträglich und wirksam zu sein. Das Molekül und seine Wirkung wurden erstmals durch Jacob NT, Lockner JVV, Kravchenko VV, Janda KD. Pharmacophore reassignment for induction of the immunosurveillance cytokine TRAIL. Angew Chem Int Ed Engl. 2014 Jun 23;-53(26):-6628-31. doi: 10.1002/anie.201402133. Epub 2014 May 18. PMID: 24838721 (Jacob et al. 2014), die sie bei der Untersuchung eines Strukturhomologons von Imipridon-201, dem Imidazolinopyrimidinon TIC10 aufgedeckt haben, dass die medizinische Wirkung zuvor fälschlich dem Strukturhomolog TIC10 zugeordnet wurde. Imipiridon-201 kann wie in Jakob et al 2014 beschrieben oder wie in EP 2698294 (Bsp. 1 und 2) beschrieben hergestellt werden.

Imipridon-206, auch bekannt als ONC206 hat die Summenformel C23H22F2N4O, die CAS-Nummer 1638178-87-6 und den IUPAC-Namen 7-Benzyl-4-(2,4-difluorobenzyl)-2,4,6,7,8,9-hexahydroimidazo[1,2-a]pyrido-[3,4-e]pyrimidin-5(1H)-on. Imipridon-206 ist ein weiteres pharmazeutisch aktives Imidazolinimipridon. Es wirkt als ein DRD2-Antagonist und ClpP-Agonist mit nanomolarer Potenz, der im Vergleich zu Imipridon-201 einen verstärkten nicht-kompetitiven DRD2-Antagonismus und eine Unterbrechung von DRD2-Homodimeren aufweist. Imipridon-206 weist ein ausgeprägtes Genexpressionsprofil sowie eine Wirksamkeit als Einzelwirkstoff und in Kombination mit Imipridon-201 in Zellen mit erworbener Resistenz gegen Imipridon-201 auf. Imipridon-206 kann gemäß dem in EP 3 068 401 B1 beschriebenen Verfahren oder dem in EP 2 698 294 B beschriebenen Verfahren mit entsprechenden Benzylen hergestellt werden.

Beide Imidazolimipridone werden in der pharmazeutischen Anwendung in Form ihres Dihydrochloridsalzes eingesetzt. Die bekannten Kristallformen weisen jedoch Nachteile hinsichtlich der Stabilität, insbesondere der Lagerfähigkeit, der Filtrierbarkeit wie auch der Kristallbildung und Fließfähigkeit auf.

In der Pharmazie ist das Auftreten von Wirkstoffen in verschiedenen kristallinen Modifikationen (Polymorphie) bei der Entwicklung von neuen Substanzen und Herstellungsverfahren ein wichtiger Aspekt. Kristalline Modifikationen einer chemischen Verbindung können dabei nicht nur im Aussehen (Kristallhabitus) und der Härte, sondern auch in zahlreichen weiteren Eigenschaften variieren. So können Unterschiede u.a. hinsichtlich der Stabilität, insbesondere der Lagerfähigkeit, der Löslichkeit, der Hygroskopizität, des Schmelzpunktes und der Feststoffdichte einen starken Einfluss auf die Qualität und die Wirksamkeit von pharmazeutischen Wirkstoffen und daraus hergestellten pharmazeutischen Zusammensetzungen nehmen.

Prozesstechnisch können Probleme wie z.B. hinsichtlich der Filtrierbarkeit, der Kristallbildung und der Fließfähigkeit auftreten. Weiter können bei der Entwicklung von Formulierungen von pharmazeutischen Zubereitungen Probleme im Hinblick auf mögliche Veränderungen der physikalisch-chemischen Eigenschaften von Wirkstoffen auftreten.

Metastabile Kristallformen können sich z.B. nachteilig auf die Stabilität bzw. Lagerungsstabilität sowie die chemisch-physikalischen Eigenschaften wie z.B. die Löslichkeit von pharmazeutischen Wirkstoffen und damit auch in pharmazeutischen Zusammensetzungen und deren Bioverfügbarkeit auswirken.

In manchen Fällen ist die technische Handhabung bekannter Kristallformen schwierig. So können nach Herstellungsverfahren des Standes der Technik bestimmte Kristallformen in Form flockiger und sehr schlecht filtrierbarer Aggregate anfallen. Weiter können Probleme mit der Kristallbildung auftreten.

Bei der Entwicklung von neuen Wirkstoffen ist es nach wie vor nicht möglich, das Auftreten sowie die mögliche Anzahl von kristallinen Modifikationen einschließlich ihrer physikalisch-chemischen Eigenschaften vorherzusagen. Die thermodynamische Stabilität als auch Unterschiede in Bezug auf biopharmazeutische Aspekte stellen dabei häufig, insbesondere in Abhängigkeit vom Applikationsweg beispielsweise bei Veränderungen der Löslichkeit, Probleme dar.

### ZUSAMMENFASSUNG DER ERFINDUNG

Die vorliegende Erfindung basiert unter anderem auf der Entwicklung neuer Herstellungsverfahren für Imipridon-201 und Imipridon-206 und deren DihydrochloridSalzen, die überraschend zu neuen röntgenographische Kristallformen führen mit verbesserten Eigenschaften wie verbesserten Verarbeitbarkeiten, verbesserten Kristallisierbarkeiten und Filtrierbarkeiten sowie erhöhten Langzeitstabilitäten im Vergleich zu den bekannten Kristallformen von Imipridon-201 und Imipridon-206, ohne einen nachteiligen Einfluss auf die Bioverfügbarkeit der daraus hergestellten Arzneiformen haben.

Folglich betrifft die Erfindung gemäß einem ersten Aspekt ein Dihydrochloridsalz von Imipridon-201 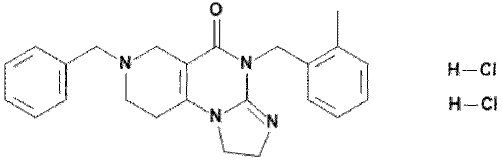 in kristalliner Form, dessen Röntgen-Pulverdiffraktogramm bei Verwendung von Cu K-α-Strahlung bei 25°C mindestens 3 der folgenden 2Θ (2 Theta)-Werte mit einer Fehlertoleranz von jeweils ± 0.2 aufweist:

| | 2Θ (2Theta)-Werte in ° |
|---|---|
| (2) | 6,9 |
| (3) | 7,7 |
| (5) | 9,6 |
| (8) | 12,9 |
| (13) | 15,5 |
| (29) | 21,2 |
| (30) | 21,4 |
| (32) | 22,5 |
| (33) | 23,1 |
| (38) | 25,3 |
| (41) | 26,8 |

Gemäß einem zweiten Aspekt betrifft die Erfindung ein Dihydrochloridsalz von Imipridon-206 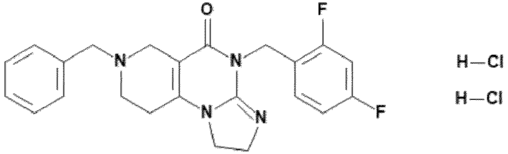 in kristalliner Form, dessen das Röntgen-Pulverdiffraktogramm bei Verwendung von Cu K-α-Strahlung bei 25°C mindestens 3 der folgenden 2Θ (2 Theta)-Werte mit einer Fehlertoleranz von jeweils ± 0.2 aufweist:

| | 2Θ (2Theta)-Werte in ° |
|---|---|
| (12) | 14,2 |
| (25) | 21,3 |
| (33) | 25,5 |
| (34) | 26,0 |
| (38) | 27,7 |

Die erfindungsgemäßen Herstellungsverfahren der Dihydrochloridsalze führen nicht nur zu erhöhten Langzeitstabilitäten der Salze, sondern stellen die Salze auch in einer besonders hohen Reinheit bereit. Zudem kommen sie nicht nur ohne das im Stand der Technik regelmäßig verwendete krebserregende Dioxan aus, sondern verwenden bevorzugt keine gesundheitsschädlichen oder umweltschädlichen Substanzen.

Gemäß einem dritten Aspekt betrifft die Anmeldung ein Verfahren zur Herstellung eines Dihydrochloridsalzes von Imipridon-201, wobei Imipridon-201 in einer sauren wässrigen Lösung gelöst und anschließend kristallisiert wird, wobei die Kristallisation durch Lösungsmittelaustausch ausgelöst wird, wobei die saure wässrige Lösung HCl enthält, und wobei das Austauschlösungsmittel, vorzugsweise ausgewählt ist aus Ketonen, Estern, Alkoholen oder Ethern, wobei das Keton bevorzugt Aceton ist.

Gemäß einem vierten Aspekt betrifft die Anmeldung ein Verfahren zur Herstellung eines Dihydrochloridsalzes von Imipridon-206, wobei Imipridon-206 in einer sauren wässrigen Lösung gelöst und anschließend kristallisiert wird, wobei die Kristallisation durch Lösungsmittelaustausch ausgelöst wird, wobei die saure wässrige Lösung HCl enthält, und wobei das Lösungsmittel vorzugsweise ausgewählt ist aus Ketonen, Estern, Alkoholen oder Ethern, wobei das Keton bevorzugt Aceton ist.

Die Dihydrochloridsalze von Imipridon-201 und Imipridon-206 können besonders dann in der gewünschten Reinheit erhalten werden, wenn die Basen von Imipridon-201 bzw. Imipridon-206 auf ein neue von den Erfindern ermittelte Weise hergestellt werden. Folglich betrifft die Erfindung nach einem fünften Aspekt ein Verfahren zur Herstellung von Imipridon-201 wie in Anspruch 14 definiert und nach einem sechsten Aspekt ein Verfahren zur Herstellung von Imipridon-206 wie in Anspruch 20 definiert.

Die Dihdyrochloridsalze sind aufgrund ihrer verbesserten Eigenschaften sehr gut für den Einsatz als Medikament, insbesondere als Krebsmittel geeignet entweder allein oder in Kombination. Folglich betrifft die Anmeldung in einem siebten Aspekt eine pharmazeutische Zusammensetzung zur Verwendung in der Behandlung von Krebs, welche ein Dihydrochlorid-Salz von Imipridon-201 gemäß einem dem ersten Aspekt enthält und/oder ein Dihydrochlorid-Salz von Imipridon-206 gemäß dem zweiten Aspekt sowie eine pharmazeutisch akzeptablen Hilfsstoff und/oder einen pharmazeutisch akzeptablen Träger.

### FIGUREN

- **Figur 1**: zeigt ein Röntgendiffraktogramm der erfindungsgemäßen Dihydrochloridsalzform von Imipridon-201.
- **Figur 2**: zeigt ein Röntgendiffraktogramm einer Dihydrochloridsalzform von Imipridon-201 aus dem Stand der Technik
- **Figur 3**: zeigt ein Röntgendiffraktogramm der erfindungsgemäßen Dihydrochloridsalzform von Imipridon-201.
- **Figur 4**: zeigt den Vergleich zweier Chromatogramm. Dabei handelt es sich um die Ergebnisse von HPLC der erfindungsgemäßen Dihydrochloridsalzform von Imipridon-201 und einem Dihydrochloridsalzform von Imipridon-201 aus dem Stand der Technik (hergestellt nach dem Verfahren gemäß EP 2 968 294 B1).
- **Figur 5**: zeigt den Vergleich zweier Chromatogramm. Dabei handelt es sich um die Ergebnisse von HPLC der erfindungsgemäßen Dihydrochloridsalzform von Imipridon-206 und einem Dihydrochloridsalzform von Imipridon-206 aus dem Stand der Technik (hergestellt nach dem Verfahren gemäß EP 2 968 294 B1 mit entsprechendem Benzylamin).
- **Figur 6**: zeigt beispielhaft ein Chromatogramm mit Ergebnistabelle für eine Charge Imipridon-201-dihydrochlorid gezeigt, die über 26 Monate bei 25°C und 60% r.F. gelagert wurde.
- **Figur 7**: zeigt beispielhaft das Chromatogramm einer Charge von Imipridon-206-dihydrochlorid, die über 24 Monate bei 25°C und 60% r.F. gelagert wurde.

### DETAILLIERTE BESCHREIBUNG DER ERFINDUNG

### Definitionen

Die "**Röntgenpulverdiffraktion"** (XRD) ist eine analytische Methode, die verwendet wird, um die kristalline Struktur von Materialien zu untersuchen. Sie basiert auf der Beugung von Röntgenstrahlen an den Atomebenen eines kristallinen Materials. Röntgenstrahlen haben eine Wellenlänge, die in der Größenordnung der Abstände zwischen Atomen in einem Kristall liegt (typischerweise 0,1 bis 0,2 nm). Wenn Röntgenstrahlen auf ein kristallines Material treffen, werden sie an den regelmäßig angeordneten Atomebenen gebeugt.

Die Beugung der Röntgenstrahlen wird durch das Bragg'sche Gesetz beschrieben: nλ=2dsin
*n*λ = 2d sin *θ*

Hierbei ist "n" eine ganze Zahl (Ordnung der Beugung), "λ" die Wellenlänge der Röntgenstrahlen, "d" der Abstand zwischen den Netzebenen im Kristall und θ der Einfallswinkel der Röntgenstrahlen. Durch die Beugung entsteht ein charakteristisches Beugungsmuster, das von der Struktur des Kristalls abhängt.

In der Röntgenpulverdiffraktion werden diese Beugungsmuster als Intensität gegen den Beugungswinkel (2θ) aufgezeichnet, was als "**Röntgenpulverdiffraktogramm"** bezeichnet wird.

Das Kristallgitter eines Feststoffes kann aus einer oder mehreren chemischen Komponenten aufgebaut sein. Daraus können neue röntgenographische Kristallformen resultieren. Daher versteht die vorliegende Erfindung unter **"röntgenographischen Kristallformen"** die mittels Röntgendiffraktometrie definierten Kristallformen von Imipridon-201-dihydrochlorid und Imipridon-206-dihydrochlorid, die im Röntgenbeugungsdiagramm durch definierte Peaks (2-Theta-Werte ±0,2°) gekennzeichnet sind und neue Eigenschaften aufweisen, wobei das Kristallgitter aus einer oder mehreren chemischen Komponenten aufgebaut sein kann. Dabei liegen den erfindungsgemäßen Imipridonen die o.g. Strukturformeln zugrunde. Die vorliegende Erfindung umfasst dabei auch Mischformen von Kristallen, in denen weitere chemische Moleküle gebunden sein können.

Unter der **"stabilen Salzform"** eines Salzes, wie beispielsweise des erfindungsgemäßen Dihydrochloridsalzes, versteht man die Kristallform, die unter gegebenen Bedingungen (Temperatur, Druck, Feuchtigkeit etc.) thermodynamisch am stabilsten ist. Diese Form zeichnet sich durch die niedrigste freie Energie im Vergleich zu anderen möglichen Kristallformen des gleichen Salzes aus.

Unter **"pharmazeutischem Wirkstoff"** versteht die vorliegende Erfindung zusätzlich zu den gesetzlichen Definitionen in den einschlägigen Regelwerken eine pharmakologisch wirksame Substanz. Synonyme sind beispielsweise "Arzneistoff" oder **"API"** (Active Pharmaceutical Ingredient).

Unter **"pharmazeutischer Zusammensetzung"** versteht die vorliegende Erfindung eine in aufwendigen Entwicklungsverfahren etablierte pharmazeutische Formulierung, die gemeinsam mit bestimmten speziell ausgewählten Hilfsstoffen zu einer Arzneiform verarbeitet wird. Die jeweilige Arzneiform wie z.B. Tablette, Kapsel, Injektionslösung etc. stellt dabei als Arzneimittel die Applikationsform dar, die auf dem jeweils definierten Weg, wie z.B. peroral oder parenteral verabreicht werden.

Unter **"Reinheit gern. ICH-Richtlinie"** versteht die vorliegende Erfindung die gemäß der ICH-Richtlinie "Impurities in new drug substances Q3A (R2)" in der derzeit gültigen Fassung festgelegten Grenzwerte. (ICH=International Conference of Harmonisation).

Unter **"Stabilität gern. ICH-Richtlinie"** versteht die vorliegende Erfindung die gemäß der ICH-Richtlinie "Stability testing of new drug substances and drug products Q1A (R2)" in der derzeit gültigen Fassung festgelegten Lagerungs- und Testbedingungen.

Ein "**Austauschlösungsmittel"** ist erfindungsgemäß ein Lösungsmittel, das in einem chemischen Prozess, insbesondere bei der Kristallisation, verwendet wird, um ein vorheriges Lösungsmittel zu ersetzen. Das Austauschlösungsmittel dient dazu, die Löslichkeit der Zielsubstanz zu verändern, um die Kristallisation zu fördern oder zu optimieren. Es wird gezielt ausgewählt, um spezifische physikalisch-chemische Eigenschaften zu nutzen, die die Trennung, Reinigung oder Gewinnung der gewünschten Substanz erleichtern. Dabei können Faktoren wie Löslichkeit, Polarität, Siedepunkt und Kompatibilität mit anderen Prozesskomponenten berücksichtigt werden. Das Austauschlösungsmittel kann sowohl organische als auch anorganische Verbindungen umfassen und ist essenziell für die Effizienz und Reinheit des kristallisierten Produkts

### Imipridon-201-dihydrochlorid

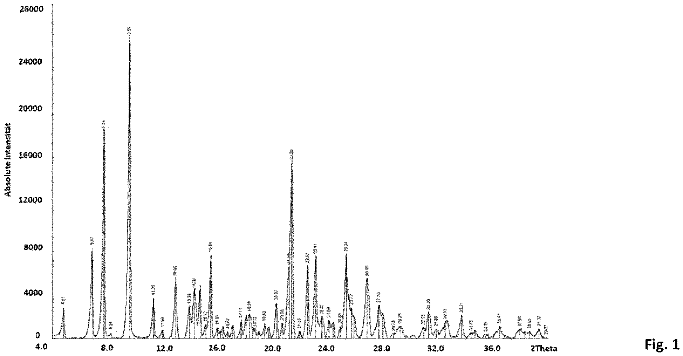

Die Erfinder haben ein neues Verfahren zur Herstellung des Dihydrochlorid-Salzes von Imipridon-201 entwickelt. Im Ergebnis entsteht dabei eine röntgenographische Kristallform des Dihydrochlorid-Salzes von Imipridon-201. Die erfindungsgemäße röntgenographische Kristallform kann durch ein Röntgenpulverdiffraktogramm charakterisiert werden wie in **Figur 1** dargestellt. Die Bestimmungsmethode und die Intensitäten der Peaks sind in Beispiel 5.1 dargestellt.

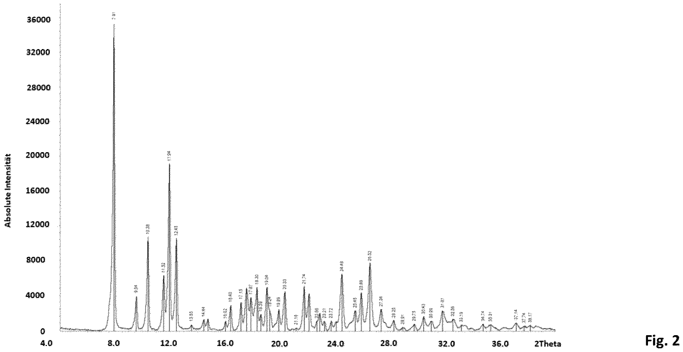

Die intensivsten und charakteristischen Signale 2Θ (2 Theta) des Röntgenpulverdiffraktogramms der röntgenographischen Kristallform von Imipridon-201 liegen bei 6,9; 7,7; 9,6; 12,9; 15,5; 21,2; 21,4; 22,5; 23,1; 25,3; und 26,8. Bezogen auf die Intensität des stärksten Peaks bei 9,6 liegt die relative Intensität dieser Peaks bei mehr als 20 %. Die Peakverteilung unterscheidet sich deutlich von der Peakverteilung von der Kristallformen gemäß dem Stand der Technik. Wie Beispiel 5.3 und insbesondere **Figur 2** zeigen, weisen die gemäß dem im Stand der Technik, z.B. in EP 2 968 294 A1, beschriebenen Herstellungsverfahren erhaltenen Salze eine gänzlich andere Peakverteilung auf. Die Erfinder haben dabei sowohl kommerziell erhältliche Imipridon-201-Proben gemessen als auch Proben, die mit dem Verfahren gemäß EP 2 968 294 A1 hergestellt wurden.

Das Dihydrochlorid-Salz von Imipridon-201 mit dieser röntgenographischen Kristallform ist dadurch gekennzeichnet, dass das Röntgenpulverdiffraktogramm dieses Salzes bei Verwendung von Cu-Kα-Strahlung bei 25°C mindestens 3 der folgenden 2Θ (2Theta)-Werte aufweist:

| | 2Θ (2Theta)-Werte in ° |
|---|---|
| (2) | 6,9 |
| (3) | 7,7 |
| (5) | 9,6 |
| (8) | 12,9 |
| (13) | 15,5 |
| (29) | 21,2 |
| (30) | 21,4 |
| (32) | 22,5 |
| (33) | 23,1 |
| (38) | 25,3 |
| (41) | 26,8 |

Das Röntgen-Pulverdiffraktogramm kann beispielsweise 3, 4, 5, 6, 7, 8, 9, oder alle 10 der genannten 2Θ (2 Theta)-Werte aufweisen. Je größer die Übereinstimmung zu dem in den Beispielen gezeigten Röntgen-Pulverdiffraktogramm, desto größer sind die erfindungsgemäßen Vorteile, insbesondere eine verbesserte Langzeitstabilität, ausgeprägt.

Darüber hinaus weist das erfindungsgemäße Dihydrochloridsalz eine verbesserte Verarbeitbarkeit, Kristallisierbarkeit, und Filtrierbarkeit auf. Durch die gute und vollständige Kristallisation resultiert eine sehr gute Filtrierbarkeit des erfindungsgemäßen Produktes und damit auch eine entsprechend hohe Ausbeute. Im Gegensatz dazu kristallisieren die Produkte des Standes der Technik sehr schlecht oder gar nicht. Es werden teilweise ölige/ pastöse Konsistenzen erhalten, die sich sehr schlecht oder gar nicht filtrieren lassen und es resultieren aufgrund der schlechten Verarbeitbarkeit entsprechend niedrige Ausbeuten

Gemäß einer Ausführungsform des Dihydrochloridsalz von Imipridon-201 weist das Röntgen-Pulverdiffraktogramm dieses Salzes bei Verwendung von Cu K-α-Strahlung bei 25°C mindestens 4 der genannten 2Θ (2 Theta)-Werte auf. Gemäß einer weiteren Ausführungsform weist es mindestens 5, mindestens 6, mindestens 7, mindestens 8, oder mindestens 9 der genannten 2Θ (2 Theta)-Werte auf.

Gemäß einer Ausführungsform weist das Röntgen-Pulverdiffraktogramm des Dihydrochloridsalzes von Imipridon-201 zusätzlich mindestens zwei der folgenden 2Θ (2 Theta)-Werte mit einer Fehlertoleranz von jeweils ± 0.2, auf: 4,8, 8,2 11,3, 12,0, 13,9, 14,3, 14,7, 15,1, 16,0, 16,2, 16,4, 16,7, 17,1, 17,7, 18,1, 18,3, 18,5, 18,7, 19,0, 19,4, 19,7, 20,3, 20,7, 22,0, 23,6, 24,1, 24,4, 24,9, 25,7, 25,9, 27,7, 28,0, 28,8, 29,2, 29,7, 31,0, 31,3, 31,9, 32,5, 33,7, 34,4, 34,7, 35,5, 36,5, 37,9, 38,3, 38,6, 39,3, 39,9. Das Röntgen-Pulverdiffraktogramm kann 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, oder sämtliche dieser Werte aufweisen. Gemäß einer Ausführungsform weist das Röntgen-Pulverdiffraktogramm des Dihydrochloridsalzes von Imipridon-201 zusätzlich mindestens vier der genannten 2Θ (2 Theta)-Werte auf. Gemäß einer Ausführungsform weist das Röntgen-Pulverdiffraktogramm des Dihydrochloridsalzes von Imipridon-201 zusätzlich mindestens sechs der genannten 2Θ (2 Theta)-Werte auf. Gemäß einer Ausführungsform weist das Röntgen-Pulverdiffraktogramm des Dihydrochloridsalzes von Imipridon-201 zusätzlich mindestens acht der genannten 2Θ (2 Theta)-Werte auf. Gemäß einer Ausführungsform weist das Röntgen-Pulverdiffraktogramm des Dihydrochloridsalzes von Imipridon-201 zusätzlich mindestens zwölf der genannten 2Θ (2 Theta)-Werte auf.

Gemäß einer Ausführungsform entspricht das Röntgenpulverdiffraktogramm des erfindungsgemäßen Dihydrochloridsalzes von Imipridon-201 bei Verwendung von Cu Kα- Strahlung bei 25 °C im Wesentlichen dem in **Figur 1** wiedergegebenen Spektrum.

Gemäß einer Ausführungsform enthält das Dihydrochloridsalz von Imipridon-201 gebundenes Wasser. Gemäß einer Ausführungsform liegt der Wassergehalt der erfindungsgemäßen röntgenographischen Kristallform von Imipridon-206-Dihydrochlorid, Bereich von 3,8 bis 7,3% für Imipridon-201-dihydrochlorid. Beispielsweise kann der Wassergehalt 3,8 %, 3,9 %, 4,0 %, 4,1 %, 4,2 %, 4,3 %, 4,4 %, 4,5 %, 4,6 %, 4,7 %, 4,8 %, 4,9 %, 5,0 %, 5,1 %, 5,2 %, 5,3 %, 5,4 %, 5,5 %, 5,6 %, 5,7 %, 5,8 %, 5,9 %, 6,0 %, 6,1 %, 6,2 %, 6,3 %, 6,4 %, 6,5 %, 6,6 %, 6,7 %, 6,8 %, 6,9 %, 7,0 %, 7,1 %, 7,2 % oder 7,3 % betragen. Gemessen wurden diese Wassergehalte mit Hilfe der Karl-Fischer Wasserbestimmung (Ofenmethode). Gemäß einer weiteren Ausführungsform weist das Infrarot-Spektrum des Dihydrochloridsalzes OH-Streckschwingungen von Wasser im Bereich von 3000 cm⁻¹ bis 3700 cm⁻¹ auf. Im Gegensatz dazu zeigen wasserfreie Kristallformen in diesem Bereich keine Signale auf.

Die röntgendiffraktometrische Überprüfung der Stabilität der erfindungsgemäßen Kristallformen zeigt ebenfalls keinerlei Veränderung über einen Zeitraum von bis zu drei Jahren. In Langzeituntersuchungen konnte bestätigt werden, dass die röntgenographisch charakteristischen Eigenschaften von Imipridon-201-dihydrochlorid in den Beugungsdiagrammen völlig stabil sind (siehe Beispiele 7 und 8).

Die vorliegende Erfindung betrifft weiterhin Verfahren zur Herstellung neuer röntgenographischer Kristallformen von Imipridon-201-dihydrochlorid. Gemäß einer Ausführungsform weist das Dihydrochlorid-Salz von Imipridon-201 eine ICH-konforme Langzeitstabilität bei 25°C und 60 % relativer Luftfeuchtigkeit von mindestens 12 Monaten auf. Beispielsweise beträgt die Langzeitstabilität bei 25°C und 60 % relativer Luftfeuchtigkeit 12 Monate, 14 Monate, 16 Monate, 18 Monate, 20 Monate, 22 Monate, 24 Monate, 26 Monate, 28 Monate, 30 Monate, 32 Monate, 34 Monate, 36 Monate, 38 Monate, 40 Monate, 42 Monate, 44 Monate, 46 Monate, oder 48 Monate. Gemäß einer weiteren Ausführungsform weist das Dihydrochlorid-Salz eine ICH-konforme Langzeitstabilität bei 25°C und 60 % relativer Luftfeuchtigkeit von mindestens 24 Monaten auf. Gemäß einer zusätzlichen Ausführungsform weist das Dihydrochlorid-Salz eine ICH-konforme Langzeitstabilität bei 25°C und 60 % relativer Luftfeuchtigkeit von mindestens 36 Monaten auf. Gemäß einer Ausführungsform stellt das erfindungsgemäße röntgenographische Dihydrochloridsalz die stabile Salzform dar.

Die erfindungsgemäße röntgenographische Kristallform ist besser geeignet zur Herstellung von (lager)stabilen Formulierungen. erfindungsgemäß

Gemäß einer Ausführungsform weist das Dihydrochlorid-Salz von Imipridon-201 eine ICH-konforme Reinheit von 99,0 bis 100% auf. Beispielsweise kann die Reinheit 99,0 %, 99,1 %, 99,2 %, 99,3 %, 99,4 %, 99,5 %, 99,6 %, 99,7 %, 99,8 %, 99,9 % oder 100 % aufweisen. Gemäß einer Ausführungsform weist das Dihydrochlorid-Salz von Imipridon-26 eine ICH-konforme Reinheit von mehr als 99,5 % auf. Gemäß einer Ausführungsform macht keine einzelne Verunreinigung gemäß ICH-Richtlinie mehr als 0,10 % aus. Einzelne Verunreinigungen machen höchstens 0,09 %, 0,08 %, 0,07 %, 0,06 %, 0,05 %, 0,04 %, 0,03 %, 0,02 %, 0,01 %, oder 0,005 % aus. Gemäß einer Ausführungsform wird die ICH-konforme Reinheit durch HPLC gemessen.

Gemäß einer Ausführungsform wird das Dihydrochlorid-Salz von Imipridon-201 zur Verwendung als Medikament eingesetzt. Gemäß einer Ausführungsform wird das Dihydrochlorid-Salz von Imipridon-201 zur Verwendung in der Behandlung von Krebs eingesetzt. Gemäß einer Ausführungsform wird das Dihydrochlorid-Salz von Imipridon-201 zur Verwendung in der Behandlung von Hirntumoren eingesetzt. Gemäß einer Ausführungsform wird das Dihydrochlorid-Salz von Imipridon-201 zur Verwendung in der Behandlung von H3 K27M Glioma eingesetzt.

### Imipridon-206-dihydrochlorid

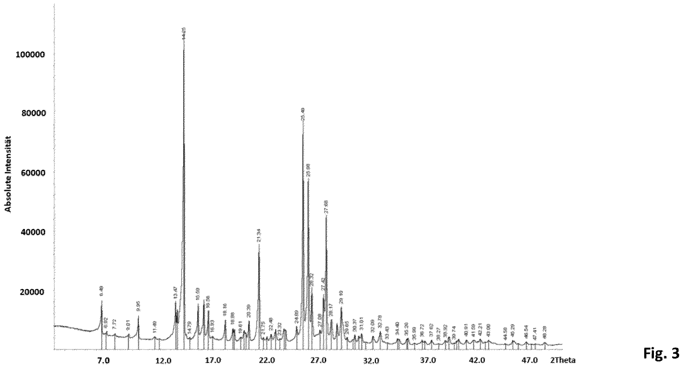

Die Erfinder haben das zur Herstellung des Dihydrochlorid-Salzes von Imipridon-201 auch für Imipridon-206 verwendete Verfahren adaptiert. Im Ergebnis entsteht dabei eine röntgenographische Kristallform des Dihydrochlorid-Salzes von Imipridon-206. Die erfindungsgemäße röntgenographische Kristallform kann durch ein Röntgenpulverdiffraktogramm charakterisiert werden wie in **Figur 3** dargestellt. Die Bestimmungsmethode und die Intensitäten der Peaks sind in Beispiel 5.3 dargestellt.

Die intensivsten und charakteristischen Signale 2Θ (2 Theta) des Röntgenpulverdiffraktogramms der röntgenographischen Kristallform von Imipridon-206 liegen bei 14,2, 21,3, 25,5, 26,0, und 27,7. Bezogen auf die Intensität des stärksten Peaks bei 14,2 liegt die relative Intensität dieser Peaks bei mehr als 20 %. Die Peakverteilung der röntgenographischen Kristallform von Imipridon-206 unterscheidet sich deutlich von der Peakverteilung von Kristallformen gemäß dem Stand der Technik. Die Erfinder haben dabei sowohl kommerziell erhältliche Imipridon-201-Proben gemessen als auch das Herstellungsverfahren gemäß EP 2 698 294 B1 durchgeführt (siehe Beispiel 6).

Das Dihydrochlorid-Salz von Imipridon-206 mit dieser röntgenographischen Kristallform ist dadurch gekennzeichnet, dass das Röntgenpulverdiffraktogramm dieses Salzes bei Verwendung von Cu-Kα-Strahlung bei 25°C mindestens 3 der folgenden 2Θ (2Theta)-Werte aufweist:

| | 2Θ (2Theta)-Werte in ° |
|---|---|
| (12) | 14,2 |
| (25) | 21,3 |
| (33) | 25,5 |
| (34) | 26,0 |
| (38) | 27,7 |

Das Röntgen-Pulverdiffraktogramm kann beispielsweise 3, 4, alle 5 der genannten 2Θ (2 Theta)-Werte aufweisen. Je größer die Übereinstimmung zum in den Beispielen gezeigten Röntgen-Pulverdiffraktogramm, desto größer sind die erfindungsgemäßen Vorteile, insbesondere eine verbesserte Langzeitstabilität, ausgeprägt.

Darüber hinaus weist das erfindungsgemäße Dihydrochloridsalz eine verbesserte Verarbeitbarkeit, Kristallisierbarkeit, und Filtrierbarkeit auf. Durch die gute und vollständige Kristallisation resultiert eine sehr gute Filtrierbarkeit des erfindungsgemäßen Produktes und damit auch eine entsprechend hohe Ausbeute. Im Gegensatz dazu kristallisieren die Produkte des Standes der Technik sehr schlecht oder gar nicht. Es werden teilweise ölige/ pastöse Konsistenzen erhalten, die sich sehr schlecht oder gar nicht filtrieren lassen und es resultieren aufgrund der schlechten Verarbeitbarkeit entsprechend niedrige Ausbeuten

Gemäß einer Ausführungsform des Dihydrochloridsalz von Imipridon-201 weist das Röntgen-Pulverdiffraktogramm dieses Salzes bei Verwendung von Cu K-α-Strahlung bei 25°C mindestens 4 der genannten 2Θ (2 Theta)-Werte auf.

Gemäß einer Ausführungsform weist das Röntgen-Pulverdiffraktogramm des Dihydrochloridsalzes von Imipridon-201 zusätzlich mindestens zwei der folgenden 2Θ (2 Theta)-Werte mit einer Fehlertoleranz von jeweils ± 0.2, auf: 6,0, 6,5, 6,9, 7,7, 9,0, 10,0, 11,5, 12,0, 13,0, 13,5, 13,6, 14,8, 15,6, 16,1, 16,6, 16,9, 18,1, 18,9, 19,0, 19,6, 19,9, 20,1, 20,4, 21,8, 22,1, 22,5, 22,9, 23,7, 23,8, 24,9, 26,3, 27,1, 27,4, 28,2, 28,7, 29,1, 29,6, 30,2, 30,4, 30,7, 31,0, 31,4, 32,1, 32,8, 33,1, 33,5, 34,4, 34,6, 35,3, 35,4, 36,0, 36,7, 37,0, 37,6, 38,3, 38,9, 39,3, 40,1, 40,9, 41,6, 41,7, 41,9, 42,2, 42,6, 43,0, 44,5, 45,3, 45,8, 46,5, 47,0, 47,4, 48,3, 49,3. Das Röntgen-Pulverdiffraktogramm kann 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, oder sämtliche dieser Werte aufweisen. Gemäß einer Ausführungsform weist das Röntgen-Pulverdiffraktogramm des Dihydrochloridsalzes von Imipridon-206 zusätzlich mindestens vier der genannten 2Θ (2 Theta)-Werte auf. Gemäß einer Ausführungsform weist das Röntgen-Pulverdiffraktogramm des Dihydrochloridsalzes von Imipridon-206 zusätzlich mindestens sechs der genannten 2Θ (2 Theta)-Werte auf. Gemäß einer Ausführungsform weist das Röntgen-Pulverdiffraktogramm des Dihydrochloridsalzes von Imipridon-206 zusätzlich mindestens acht der genannten 2Θ (2 Theta)-Werte auf. Gemäß einer Ausführungsform weist das Röntgen-Pulverdiffraktogramm des Dihydrochloridsalzes von Imipridon-206 zusätzlich mindestens zwölf der genannten 2Θ (2 Theta)-Werte auf.

Gemäß einer Ausführungsform entspricht das Röntgenpulverdiffraktogramm des erfindungsgemäßen Dihydrochloridsalzes von Imipridon-206 bei Verwendung von Cu Kα- Strahlung bei 25 °C im Wesentlichen dem in **Figur 3** wiedergegebenen Spektrum.

Gemäß einer Ausführungsform enthält das Dihydrochloridsalz von Imipridon-206 gebundenes Wasser. Gemäß einer Ausführungsform liegt der Wassergehalt der erfindungsgemäßen röntgenographischen Kristallform von Imipridon-206-Dihydrochlorid, Bereich von 3,6 bis 7,0 % für Imipridon-201-dihydrochlorid. Beispielsweise kann der Wassergehalt 3,6 %, 3,7 %, 3,8 %, 3,9 %, 4,0 %, 4,1 %, 4,2 %, 4,3 %, 4,4 %, 4,5 %, 4,6 %, 4,7 %, 4,8 %, 4,9 %, 5,0 %, 5,1 %, 5,2 %, 5,3 %, 5,4 %, 5,5 %, 5,6 %, 5,7 %, 5,8 %, 5,9 %, 6,0 %, 6,1 %, 6,2 %, 6,3 %, 6,4 %, 6,5 %, 6,6 %, 6,7 %, 6,8 %, 6,9 %, oder 7,0 % betragen. Gemessen wurden diese Wassergehalte mit Hilfe der Karl-Fischer Wasserbestimmung (Ofenmethode).

Die röntgendiffraktometrische Überprüfung der Stabilität der erfindungsgemäßen Kristallformen zeigt ebenfalls keinerlei Veränderung über einen Zeitraum von bis zu drei Jahren. In Langzeituntersuchungen konnte bestätigt werden, dass die röntgenographisch charakteristischen Eigenschaften von Imipridon-206-dihydrochlorid in den Beugungsdiagrammen völlig stabil sind. Die vorliegende Erfindung betrifft weiterhin Verfahren zur Herstellung neuer röntgenographischer Kristallformen von Imipridon-206-dihydrochlorid. Gemäß einer Ausführungsform weist das Dihydrochlorid-Salz von Imipridon-206 eine ICH-konforme Langzeitstabilität bei 25°C und 60 % relativer Luftfeuchtigkeit von mindestens 12 Monaten auf. Beispielsweise beträgt die Langzeitstabilität bei 25°C und 60 % relativer Luftfeuchtigkeit 12 Monate, 14 Monate, 16 Monate, 18 Monate, 20 Monate, 22 Monate, 24 Monate, 26 Monate, 28 Monate, 30 Monate, 32 Monate, 34 Monate, 36 Monate, 38 Monate, 40 Monate, 42 Monate, 44 Monate, 46 Monate, oder 48 Monate. Gemäß einer weiteren Ausführungsform weist das Dihydrochlorid-Salz eine ICH-konforme Langzeitstabilität bei 25°C und 60 % relativer Luftfeuchtigkeit von mindestens 24 Monaten auf. Gemäß einer zusätzlichen Ausführungsform weist das Dihydrochlorid-Salz eine ICH-konforme Langzeitstabilität bei 25°C und 60 % relativer Luftfeuchtigkeit von mindestens 36 Monaten auf. Gemäß einer Ausführungsform stellt das erfindungsgemäße röntgenographische Dihydrochloridsalz die stabile Salzform dar.

Gemäß einer Ausführungsform weist das Dihydrochlorid-Salz von Imipridon-206 eine ICH-konforme Reinheit von mehr als 99,0 % auf. Beispielsweise kann die Reinheit 99,0 %, 99,1 %, 99,2 %, 99,3 %, 99,4 %, 99,5 %, 99,6 %, 99,7 %, 99,8 %, 99,9 % oder 100 % aufweisen. Gemäß einer Ausführungsform weist das Dihydrochlorid-Salz von Imipridon-26 eine ICH-konforme Reinheit von mehr als 99,5 % auf. Gemäß einer Ausführungsform macht keine einzelne Verunreinigung gem. ICH-Richtlinie mehr als 0,1 % aus. Einzelne Verunreinigungen machen höchstens 0,09 %, 0,08 %, 0,07 %, 0,06 %, 0,05 %, 0,04 %, 0,03 %, 0,02 %, 0,01 %, oder 0,005 % aus. Gemäß einer Ausführungsform wird die ICH-konforme Reinheit durch HPLC gemessen.

Die erfindungsgemäßen röntgenographischen Kristallformen von Imipridon-201-dihydrochlorid und Imipridon-206-dihydrochlorid sind besser geeignet zur Herstellung von (lager)stabilen Formulierungen als die Kristallformen von Imipridon-201-dihydrochlorid und Imipridon-206-dihydrochlorid aus dem Stand der Technik.

Die erfindungsgemäßen röntgenographischen Kristallformen von Imipridon-201-dihydrochlorid und Imipridon-206-dihydrochlorid sind nicht hygroskopisch und hinsichtlich ihrer Kristallform und Kristallstruktur als auch der chemischen Reinheit lagerstabil, sowohl als Wirkstoff allein als auch in pharmazeutischen Zusammensetzungen in Mischungen mit Hilfsstoffen.

Es ist bekannt, dass sich metastabile Kristallformen in entsprechend stabilere Formen umwandeln können. Die thermodynamisch stabilere Form entsteht z.B. bei längerer Lagerung in festem Zustand, durch Rühren in Suspension oder durch Kristallisation bei anderen Bedingungen, wie Temperatur, Konzentration oder unterschiedlichen Wasseranteilen im Lösungsmittel. Ein möglicher Einfluss der Luftfeuchtigkeit auf die Bildung und Stabilisierung von röntgenographischen Kristallformen ist ebenfalls bekannt. Insbesondere hat sich das Screening durch Suspendieren in verschiedenen Lösungsmitteln als Verfahren zur Bestimmung der stabilsten Form etabliert.

So haben eigene Untersuchungen gezeigt, dass eine Umwandlung von nach dem Stand der Technik hergestellten Produkten (z-B. gemäß EP 2 698 294, Bsp. 1 und 2) durch Suspendieren mit dem erfindungsgemäßen Lösungsmittel in das erfindungsgemäße Imipridon-201-dihydrochlorid möglich ist. Damit wird der Nachweis erbracht, dass die erfindungsgemäß hergestellte Verbindung die entsprechend stabilere Form ist.

Gemäß einer Ausführungsform wird das Dihydrochlorid-Salz von Imipridon-206 zur Verwendung als Medikament eingesetzt. Gemäß einer Ausführungsform wird das Dihydrochlorid-Salz von Imipridon-206 zur Verwendung in der Behandlung von Krebs eingesetzt. Gemäß einer Ausführungsform wird das Dihydrochlorid-Salz von Imipridon-206 zur Verwendung in der Behandlung von Tumoren des zentralen Nervensystems und/oder Hirntumoren eingesetzt.

### Verfahren zur Herstellung von Imipridon-201-dihydrochlorid

Die vorliegende Erfindung überwindet die Nachteile des Standes der Technik auch dadurch, dass sie auf hochtoxische, kanzerogene und umweltschädliche Lösungsmittel wie z.B. Dioxan vollständig verzichtet. Dioxan ist in der Kandidatenliste der besonders besorgniserregenden Stoffe (SVHC) gemäß Artikel 59 Absatz 10 der Verordnung (EG) Nr. 1907/2006 (REACH) enthalten. Dies gilt hinsichtlich der Toxizität für den menschlichen Organismus als auch für die Umwelttoxizität.

Gemäß einem dritten Aspekt betrifft die Anmeldung ein Verfahren zur Herstellung eines Dihydrochloridsalzes von Imipridon-201. In diesem Verfahren wird wie im Stand der Technik Imipridon-201 in einer sauren wässrigen Lösung gelöst, wobei die saure wässrige Lösung HCl enthält, und anschließend kristallisiert. Allerdings wird die Kristallisation durch Lösungsmittelaustausch ausgelöst. Dabei wird ein Austauschlösungsmittel eingesetzt, das aus Ketonen, Estern, Alkoholen und Ethern ausgewählt sein kann. Gemäß einer Ausführungsform ist das Austauschlösungsmittel ein Keton. Gemäß einer bevorzugten Ausführungsform ist das Keton Aceton.

Gemäß einer Ausführungsform wird das Verfahren ohne Dioxan durchgeführt. Bevorzugt wird das Verfahren ohne Lösungsmittel durchgeführt, die toxisch, krebserregend und/oder umweltschädlich sind.

Gemäß einer Ausführungsform ist umfasst das Verfahren den Schritt a) Erwärmen einer HCl-haltigen wässrigen Lösung. Dabei kann beispielsweise 0,5 ml, 1 ml, 1,5 ml, 2 ml, 2,5 ml, 3 ml, 3,5 ml, 4 ml, 4,5 ml, 5 ml, 5,5 ml, 6 ml, 6,5 ml, 7 ml, 7,5 ml, oder 8 ml HCl-haltiger wässrige Lösung pro 1 g Imipridon-201 erwärmt. Gemäß einer Ausführungsform wird 0,5 bis 5 ml HCl-haltige wässrige Lösung pro 1 g Imipridon-201 erwärmt. Gemäß einer weiteren Ausführungsform wird 1 bis 3 ml HCl-haltige wässrige Lösung pro 1 g Imipridon-201 erwärmt.

Gemäß einer Ausführungsform wird die HCl-haltige wässrige Lösung auf eine Temperatur im Bereich von 50-70°C erhitzt. Beispielsweise kann die Temperatur 50°C, 52°C, 54°C, 56°C, 58°C, 60°C, 62°C, 64°C, 66°C, 68°C oder 70°C betragen."

In Schritt b) wird Imipridon-201 zugegeben.

Gemäß einer Ausführungsform umfasst das Verfahren den Schritt c) Einstellung des pH-Wertes der Mischung auf einen Wert im Bereich von 3 bis 5. Beispielsweise kann der pH-Wert 3,0, 3,1, 3,2, 3,3, 3,4, 3,5, 3,6, 3,7, 3,8, 3,9, 4,0, 4,1, 4,2, 4,3, 4,4, 4,5, 4,6, 4,7, 4,8, 4,9 oder 5,0 betragen. Gemäß einer weiteren Ausführungsform wird der pH-Wert der Mischung auf einen Wert im Bereich von 3,5 bis 4,5 eingestellt. Besonders bevorzugt erfolgt die Einstellung des pH-Wertes durch Zutropfen weiterer HCl-haltiger wässriger Lösung.

Gemäß einer optionalen Ausführungsform umfasst das Verfahren den Schritt d) Filtration der Lösung. Bevorzugt erfolgt die Filtration der Lösung mit einer Porengröße von höchstens 1 µm. Besonders bevorzugt erfolgt die Filtration der Lösung mit einer Porengröße von höchstens 0,5 µm.

Gemäß einer Ausführungsform umfasst das Verfahren den Schritt e) Einstellen der Temperatur auf einen Wert im Bereich von 30 bis 70 °C. Beispielsweise kann die Temperatur 30 °C, 32 °C, 34 °C, 36 °C, 38 °C, 40 °C, 42 °C, 44 °C, 46 °C, 48 °C, 50 °C, 52 °C, 54 °C, 56 °C, 58 °C, 60 °C, 62 °C, 64 °C, 66 °C, 68 °C oder 70 °C betragen. Gemäß einer weiteren Ausführungsform wird die Temperatur auf einen Wert im Bereich von 40 bis 60 °C eingestellt.

Gemäß einer Ausführungsform umfasst das Verfahren den Schritt f) unter Rühren tropfenweise Zugabe von 10%iger HCl bis zu einem pH-Wert im Bereich von 0,4 bis 2,0. Beispielsweise kann der pH-Wert 0,4, 0,5, 0,6, 0,7, 0,8, 0,9, 1,0, 1,1, 1,2, 1,3, 1,4, 1,5, 1,6, 1,7, 1,8, 1,9 oder 2,0 betragen. Gemäß einer weiteren Ausführungsform wird der pH-Wert im Bereich von 0,6 bis 1,5 eingestellt. Gemäß einer zusätzlichen Ausführungsform wird der pH-Wert im Bereich von 0,8 bis 1,0 eingestellt.

Gemäß einer Ausführungsform umfasst das Verfahren den Schritt g) Zugabe des Austauschlösungsmittels. Beispielsweise können 5 ml, 6 ml, 7 ml, 8 ml, 9 ml, 10 ml, 11 ml, 12 ml, 13 ml, 14 ml oder 15 ml Austauschlösungsmittel pro 1 g Imipridon-201 zugegeben werden. Gemäß einer weiteren Ausführungsform werden 5 bis 15 ml Austauschlösungsmittel pro 1 g Imipridon-201 zugegeben. Gemäß einer zusätzlichen Ausführungsform werden 8 bis 12 ml Austauschlösungsmittel pro 1 g Imipridon-201 zugegeben.

Gemäß einer Ausführungsform umfasst das Verfahren den Schritt h) Abkühlen auf eine Temperatur im Bereich von 10 bis 30 °C. Beispielsweise kann die Temperatur 10 °C, 12 °C, 14 °C, 16 °C, 18 °C, 20 °C, 22 °C, 24 °C, 26 °C, 28 °C oder 30 °C betragen. Gemäß einer weiteren Ausführungsform wird die Temperatur im Bereich von 15 bis 25 °C eingestellt. Gemäß einer zusätzlichen Ausführungsform wird die Temperatur auf ungefähr Raumtemperatur abgekühlt.

Gemäß einer Ausführungsform umfasst das Verfahren den Schritt i) Zugabe einer weiteren Menge des Austauschlösungsmittels. Beispielsweise können 30 ml, 32 ml, 34 ml, 36 ml, 38 ml, 40 ml, 42 ml, 44 ml, 46 ml, 48 ml oder 50 ml Lösungsmittel pro 1 g Imipridon-201 zugegeben werden. Gemäß einer weiteren Ausführungsform werden 30 bis 50 ml Lösungsmittel pro 1 g Imipridon-201 zugegeben. Gemäß einer zusätzlichen Ausführungsform erfolgt die Zugabe über einen Zeitraum von mindestens 2 Stunden. Besonders bevorzugt erfolgt die Zugabe über einen Zeitraum von mindestens 4 Stunden.

Gemäß einer Ausführungsform umfasst das Verfahren den Schritt j) Rühren der Lösung für mindestens 0,5 Stunden. Gemäß einer weiteren Ausführungsform wird die Lösung für mindestens 1,0 Stunden bei Raumtemperatur gerührt.

Gemäß einer Ausführungsform umfasst das Verfahren den Schritt k) Abtrennung des Präzipitats. Insbesondere erfolgt die Abtrennung bevorzugt durch Filtration. Besonders bevorzugt erfolgt die Filtration mit einer Porengröße im Bereich von 10 bis 100 µm. Beispielsweise kann die Porengröße 10 µm, 20 µm, 30 µm, 40 µm, 50 µm, 60 µm, 70 µm, 80 µm, 90 µm oder 100 µm betragen. Gemäß einer weiteren Ausführungsform erfolgt die Filtration mit einer Porengröße im Bereich von 16 bis 40 µm.

Gemäß einer Ausführungsform umfasst das Verfahren den Schritt I) Waschen des Präzipitats mit 1,0 bis 10,0 ml des Austauschlösungsmittels. Beispielsweise kann das Präzipitat mit 1,0 ml, 2,0 ml, 3,0 ml, 4,0 ml, 5,0 ml, 6,0 ml, 7,0 ml, 8,0 ml, 9,0 ml oder 10,0 ml des Austauschlösungsmittels gewaschen werden. Gemäß einer weiteren Ausführungsform wird das Präzipitat mit 2,0 bis 7,0 ml des Austauschlösungsmittels gewaschen. Gemäß einer zusätzlichen Ausführungsform wird das Präzipitat mit 3,0 bis 5,0 ml des Austauschlösungsmittels gewaschen.

Gemäß einer Ausführungsform umfasst das Verfahren den Schritt m) Trocknung des Präzipitats, wobei die Trocknung über einen Zeitraum von mindestens 10 Stunden stattfindet. Gemäß einer weiteren Ausführungsform erfolgt die Trocknung über einen Zeitraum von mindestens 14 Stunden. Gemäß einer zusätzlichen Ausführungsform erfolgt die Trocknung über einen Zeitraum von mindestens 16 Stunden.

Die Temperatur während der Trocknung liegt gemäß einer Ausführungsform im Bereich von 30 bis 70 °C. Beispielsweise kann die Temperatur 30 °C, 35 °C, 40 °C, 45 °C, 50 °C, 55 °C, 60 °C, 65 °C oder 70 °C betragen. Gemäß einer weiteren Ausführungsform liegt die Temperatur im Bereich von 40 bis 60 °C. Insbesondere liegt die Temperatur gemäß einer Ausführungsform im Bereich von 45 bis 55 °C.

Der Druck während der Trocknung liegt gemäß einer Ausführungsform im Bereich von 10 bis 100 mbar. Beispielsweise kann der Druck 10 mbar, 20 mbar, 30 mbar, 40 mbar, 50 mbar, 60 mbar, 70 mbar, 80 mbar, 90 mbar oder 100 mbar betragen. Gemäß einer weiteren Ausführungsform liegt der Druck im Bereich von 20 bis 40 mbar.

Gemäß einer Ausführungsform des Schritts a) enthält die HCl-haltige wässrige Lösung eine 10 % HCl-Lösung im Bereich von 10 bis 40 Vol.%. Beispielsweise kann die HCl-Lösung 10 Vol.%, 15 Vol.%, 20 Vol.%, 25 Vol.%, 30 Vol.%, 35 Vol.% oder 40 Vol.% betragen. Gemäß einer weiteren Ausführungsform enthält die HCl-Lösung im Bereich von 15 bis 30 Vol.%. Gemäß einer zusätzlichen Ausführungsform enthält die HCl-Lösung im Bereich von 20 bis 25 Vol.%.

Das Verfahren kann noch durch die Schritte n) bis t) ergänzt werden. Damit wird eine höhere Reinheit des Produkts erreicht. Gemäß einer Ausführungsform umfasst das Verfahren den Schritt n) Zugabe des Präzipitats zu einem Alkohol. Der Alkohol ist bevorzugt ausgewählt aus der Gruppe der Alkohole mit 1 bis 4 C-Atomen. Es kann sich somit um Methanol, Ethanol, 1-Propanol, 2-Propanol, 1-Butanol, 2-Butanol, 2-Methyl-1-propanol oder 2-Methyl-2-propanol handeln. Bevorzugt wird Ethanol verwendet. Besonders bevorzugt wird 96 %iger Ethanol (EtOH 96 %) verwendet.

Gemäß einer Ausführungsform werden 2 bis 40 ml des Alkohols pro 1 g des Präzipitats verwendet. Beispielsweise können 2 ml, 5 ml, 10 ml, 15 ml, 20 ml, 25 ml, 30 ml, 35 ml oder 40 ml des Alkohols pro 1 g des Präzipitats verwendet werden. Gemäß einer weiteren Ausführungsform werden 10 bis 30 ml des Alkohols pro 1 g des Präzipitats verwendet. Gemäß einer zusätzlichen Ausführungsform werden 15 bis 20 ml des Alkohols pro 1 g des Präzipitats verwendet.

Gemäß einer Ausführungsform umfasst das Verfahren den Schritt o) Erhitzen der Mischung unter Rühren mit einer Wärmequelle auf eine Temperatur im Bereich von 50 bis 95 °C. Beispielsweise kann die Temperatur 50 °C, 55 °C, 60 °C, 65 °C, 70 °C, 75 °C, 80 °C, 85 °C, 90 °C oder 95 °C betragen. Gemäß einer weiteren Ausführungsform wird die Temperatur im Bereich von 60 bis 90 °C eingestellt. Gemäß einer zusätzlichen Ausführungsform wird die Temperatur im Bereich von 70 bis 80 °C eingestellt. Bevorzugt erfolgt das Erhitzen so, dass das verdampfende Ethanol rückfließt.

Gemäß einer Ausführungsform umfasst das Verfahren den Schritt p) Abkühlen auf eine Temperatur im Bereich von 20 bis 40 °C über einen Zeitraum von mindestens 1 Stunde. Beispielsweise kann die Temperatur 20 °C, 25 °C, 30 °C, 35 °C oder 40 °C betragen. Gemäß einer weiteren Ausführungsform wird die Temperatur im Bereich von 25 bis 35 °C eingestellt.

Gemäß einer zusätzlichen Ausführungsform erfolgt das Abkühlen über einen Zeitraum von mindestens 1,5 Stunden. Besonders bevorzugt erfolgt das Abkühlen über einen Zeitraum von mindestens 2,5 Stunden.

Gemäß einer Ausführungsform umfasst das Verfahren den Schritt q) Abtrennung des Präzipitats. Bevorzugt erfolgt die Abtrennung durch Filtration. Besonders bevorzugt erfolgt die Filtration mit einer Porengröße von höchstens 1 µm.

Gemäß einer optionalen Ausführungsform umfasst das Verfahren den Schritt r) Waschen des Präzipitats mit 0,1 bis 5 ml des Alkohols pro 1 g des Präzipitats. Beispielsweise kann das Präzipitat mit 0,1 ml, 0,5 ml, 1 ml, 1,5 ml, 2 ml, 2,5 ml, 3 ml, 3,5 ml, 4 ml, 4,5 ml oder 5 ml des Alkohols pro 1 g des Präzipitats gewaschen werden.

Gemäß einer weiteren Ausführungsform wird das Präzipitat mit 1 bis 3 ml des Alkohols pro 1 g des Präzipitats gewaschen. Gemäß einer zusätzlichen Ausführungsform wird das Präzipitat mit 1,5 bis 2,5 ml des Alkohols pro 1 g des Präzipitats gewaschen.

Gemäß einer Ausführungsform umfasst das Verfahren den Schritt s) Waschen des Präzipitats mit 1,0 bis 8,0 ml eines organischen Lösungsmittels, ausgewählt aus Ketonen, Estern, Alkoholen oder Ethern. Beispielsweise kann das Präzipitat mit 1,0 ml, 2,0 ml, 3,0 ml, 4,0 ml, 5,0 ml, 6,0 ml, 7,0 ml oder 8,0 ml des organischen Lösungsmittels gewaschen werden. Gemäß einer weiteren Ausführungsform wird das Präzipitat mit 2,0 bis 6,0 ml des organischen Lösungsmittels gewaschen. Gemäß einer zusätzlichen Ausführungsform wird das Präzipitat mit 3,5 bis 4,5 ml des organischen Lösungsmittels gewaschen. Das organische Lösungsmittel ist bevorzugt ein Keton, wobei Aceton besonders bevorzugt ist.

Gemäß einer Ausführungsform umfasst das Verfahren den Schritt t) Trocknung des Feststoffs, wobei die Trocknung einen oder mehrere Trocknungsschritte aufweist. Bevorzugt ist mindestens ein Trocknungsschritt eine Vakuum-Trocknung über einen Zeitraum von 1 bis 5 Tagen. Beispielsweise kann der Trocknungsschritt 1 Tag, 2 Tage, 3 Tage, 4 Tage oder 5 Tage betragen. Gemäß einer weiteren Ausführungsform erfolgt die Vakuum-Trocknung über einen Zeitraum von 1,5 bis 4 Tagen.

Die Temperatur während der Vakuum-Trocknung liegt gemäß einer Ausführungsform im Bereich von 50 bis 100 °C. Beispielsweise kann die Temperatur 50 °C, 60 °C, 70 °C, 80 °C, 90 °C oder 100 °C betragen. Gemäß einer weiteren Ausführungsform liegt die Temperatur im Bereich von 65 bis 95 °C. Insbesondere liegt die Temperatur gemäß einer Ausführungsform im Bereich von 75 bis 85 °C.

### Verfahren zur Herstellung von Imipridon-206-dihydrochlorid

Gemäß einem dritten Aspekt betrifft die Anmeldung ein Verfahren zur Herstellung eines Dihydrochloridsalzes von Imipridon-206. In diesem Verfahren wird wie im Stand der Technik Imipridon-206 in einer sauren wässrigen Lösung gelöst, wobei die saure wässrige Lösung HCl enthält, und anschließend kristallisiert. Allerdings wird die Kristallisation durch Lösungsmittelaustausch ausgelöst. Dabei wird ein Austauschlösungsmittel eingesetzt, das aus Ketonen, Estern, Alkoholen und Ethern ausgewählt sein kann. Gemäß einer Ausführungsform ist das Austauschlösungsmittel ein Keton. Gemäß einer bevorzugten Ausführungsform ist das Keton Aceton.

Gemäß einer Ausführungsform wird das Verfahren ohne Dioxan durchgeführt. Bevorzugt wird das Verfahren ohne Lösungsmittel durchgeführt, die toxisch, krebserregend und/oder umweltschädlich sind.

Gemäß einer Ausführungsform umfasst das Verfahren den Schritt a) Erwärmen einer HCl-haltigen wässrigen Lösung. Beispielsweise können 0,5 ml, 1 ml, 1,5 ml, 2 ml, 2,5 ml, 3 ml, 3,5 ml, 4 ml, 4,5 ml oder 5 ml HCl-haltiger wässriger Lösung pro 1 g Imipridon-206 verwendet werden. Gemäß einer weiteren Ausführungsform werden 1 bis 3 ml HCl-haltige wässrige Lösung pro 1 g Imipridon-206 verwendet. Die HCl-haltige wässrige Lösung wird vorzugsweise auf eine Temperatur im Bereich von 55 bis 65 °C erhitzt.

Gemäß einer Ausführungsform umfasst das Verfahren den Schritt b) Zugabe von Imipridon-206.

Gemäß einer Ausführungsform umfasst das Verfahren den Schritt c) Einstellen des pH-Wertes der Mischung auf einen Wert im Bereich von 3 bis 5. Beispielsweise kann der pH-Wert 3,0, 3,5, 4,0, 4,5 oder 5,0 betragen. Gemäß einer weiteren Ausführungsform wird der pH-Wert auf einen Wert im Bereich von 3,5 bis 4,5 eingestellt. Besonders bevorzugt erfolgt die Einstellung des pH-Wertes durch Zutropfen weiterer HCl-haltiger wässriger Lösung."

Gemäß einer optionalen Ausführungsform umfasst das Verfahren den Schritt d) Filtration der Lösung. Bevorzugt erfolgt die Filtration der Lösung mit einer Porengröße von höchstens 1 µm. Besonders bevorzugt erfolgt die Filtration der Lösung mit einer Porengröße von höchstens 0,5 µm.

Gemäß einer Ausführungsform umfasst das Verfahren den Schritt e) Einstellen der Temperatur auf einen Wert im Bereich von 30 bis 70 °C. Beispielsweise kann die Temperatur 30 °C, 40 °C, 50 °C, 60 °C oder 70 °C betragen. Gemäß einer weiteren Ausführungsform wird die Temperatur im Bereich von 40 bis 60 °C eingestellt.

Gemäß einer Ausführungsform umfasst das Verfahren den Schritt f) unter Rühren tropfenweise Zugabe von 10%iger HCl bis zu einem pH-Wert im Bereich von 0,5 bis 3,0. Beispielsweise kann der pH-Wert 0,5,1,0,1,5, 2,0, 2,5 oder 3,0 betragen. Gemäß einer weiteren Ausführungsform wird der pH-Wert im Bereich von 1,0 bis 1,8 eingestellt. Insbesondere wird der pH-Wert im Bereich von 1,3 bis 1,5 eingestellt. Gemäß einer Ausführungsform umfasst das Verfahren den Schritt g) Zugabe des organischen Lösungsmittels. Beispielsweise können 5 ml, 6 ml, 7 ml, 8 ml, 9 ml, 10 ml, 11 ml, 12 ml, 13 ml, 14 ml oder 15 ml Lösungsmittel pro 1 g Imipridon-206 verwendet werden. Gemäß einer weiteren Ausführungsform werden 8 bis 12 ml Lösungsmittel pro 1 g Imipridon-206 verwendet.

Gemäß einer Ausführungsform umfasst das Verfahren den Schritt h) Abkühlen auf eine Temperatur im Bereich von 10 bis 30 °C. Beispielsweise kann die Temperatur 10 °C, 12 °C, 14 °C, 16 °C, 18 °C, 20 °C, 22 °C, 24 °C, 26 °C, 28 °C oder 30 °C betragen. Gemäß einer weiteren Ausführungsform wird die Temperatur im Bereich von 15 bis 25 °C eingestellt. Gemäß einer zusätzlichen Ausführungsform wird die Temperatur auf ungefähr Raumtemperatur abgekühlt.

Gemäß einer Ausführungsform umfasst das Verfahren den Schritt i) Zugabe einer weiteren Menge des organischen Lösungsmittels. Beispielsweise können 30 ml, 32 ml, 34 ml, 36 ml, 38 ml, 40 ml, 42 ml, 44 ml, 46 ml, 48 ml oder 50 ml Lösungsmittel pro 1 g Imipridon-206 verwendet werden. Die Zugabe erfolgt vorzugsweise über einen Zeitraum von mindestens 2 Stunden. Beispielsweise kann der Zeitraum 2 Stunden, 3 Stunden, 4 Stunden oder mehr betragen. Gemäß einer weiteren Ausführungsform erfolgt die Zugabe über einen Zeitraum von mindestens 4 Stunden.

Gemäß einer Ausführungsform umfasst das Verfahren den Schritt j) Rühren der Lösung für mindestens 0,5 Stunden. Gemäß einer weiteren Ausführungsform wird die Lösung für mindestens 1,0 Stunden bei Raumtemperatur gerührt.

Gemäß einer Ausführungsform umfasst das Verfahren den Schritt k) Abtrennung des Präzipitats. Insbesondere erfolgt die Abtrennung bevorzugt durch Filtration. Besonders bevorzugt erfolgt die Filtration mit einer Porengröße im Bereich von 10 bis 100 µm. Beispielsweise kann die Porengröße 10 µm, 20 µm, 30 µm, 40 µm, 50 µm, 60 µm, 70 µm, 80 µm, 90 µm oder 100 µm betragen. Gemäß einer weiteren Ausführungsform erfolgt die Filtration mit einer Porengröße im Bereich von 16 bis 40 µm.

Gemäß einer Ausführungsform umfasst das Verfahren den Schritt I) Waschen des Präzipitats mit 1,0 bis 10,0 ml des Austauschlösungsmittels. Beispielsweise kann das Präzipitat mit 1,0 ml, 2,0 ml, 3,0 ml, 4,0 ml, 5,0 ml, 6,0 ml, 7,0 ml, 8,0 ml, 9,0 ml oder 10,0 ml des Austauschlösungsmittels gewaschen werden. Gemäß einer weiteren Ausführungsform wird das Präzipitat mit 2,0 bis 7,0 ml des Austauschlösungsmittels gewaschen. Gemäß einer zusätzlichen Ausführungsform wird das Präzipitat mit 3,0 bis 5,0 ml des Austauschlösungsmittels gewaschen.

Gemäß einer Ausführungsform umfasst das Verfahren den Schritt m) Trocknung des Präzipitats, wobei die Trocknung über einen Zeitraum von mindestens 10 Stunden stattfindet. Gemäß einer weiteren Ausführungsform erfolgt die Trocknung über einen Zeitraum von mindestens 14 Stunden. Gemäß einer zusätzlichen Ausführungsform erfolgt die Trocknung über einen Zeitraum von mindestens 16 Stunden.

Die Temperatur während der Trocknung liegt gemäß einer Ausführungsform im Bereich von 30 bis 70 °C. Beispielsweise kann die Temperatur 30 °C, 35 °C, 40 °C, 45 °C, 50 °C, 55 °C, 60 °C, 65 °C oder 70 °C betragen. Gemäß einer weiteren Ausführungsform liegt die Temperatur im Bereich von 40 bis 60 °C. Insbesondere liegt die Temperatur gemäß einer Ausführungsform im Bereich von 45 bis 55 °C.

Der Druck während der Trocknung liegt gemäß einer Ausführungsform im Bereich von 10 bis 100 mbar. Beispielsweise kann der Druck 10 mbar, 20 mbar, 30 mbar, 40 mbar, 50 mbar, 60 mbar, 70 mbar, 80 mbar, 90 mbar oder 100 mbar betragen. Gemäß einer weiteren Ausführungsform liegt der Druck im Bereich von 20 bis 40 mbar.

Gemäß einer Ausführungsform des Schritts a) enthält die HCl-haltige wässrige Lösung eine 10 % HCl-Lösung im Bereich von 10 bis 40 Vol.%. Beispielsweise kann die HCl-Lösung 10 Vol.%, 15 Vol.%, 20 Vol.%, 25 Vol.%, 30 Vol.%, 35 Vol.% oder 40 Vol.% betragen. Gemäß einer weiteren Ausführungsform enthält die HCl-Lösung im Bereich von 15 bis 30 Vol.%. Gemäß einer zusätzlichen Ausführungsform enthält die HCl-Lösung im Bereich von 20 bis 25 Vol.%.

Das Verfahren kann noch durch die Schritte n) bis t) ergänzt werden. Gemäß einer Ausführungsform umfasst das Verfahren den Schritt n) Zugabe des Präzipitats zu einem Alkohol, ausgewählt aus der Gruppe der Alkohole mit 1 bis 4 C-Atomen. Bevorzugt wird Ethanol verwendet. Besonders bevorzugt wird 96 %iger Ethanol (EtOH 96 %) verwendet. Beispielsweise können 1 ml, 2 ml, 3 ml, 4 ml, 5 ml, 6 ml, 7 ml, 8 ml, 9 ml, 10 ml, 11 ml, 12 ml, 13 ml, 14 ml, 15 ml, 16 ml, 17 ml, 18 ml, 19 ml, 20 ml, 21 ml, 22 ml, 23 ml, 24 ml oder 25 ml des Alkohols pro 1 g des Präzipitats verwendet werden. Gemäß einer weiteren Ausführungsform werden 2 bis 15 ml des Alkohols pro 1 g des Präzipitats verwendet. Insbesondere werden 4 bis 8 ml des Alkohols pro 1 g des Präzipitats verwendet. Gemäß einer optionalen Ausführungsform umfasst das Verfahren den Schritt o) weitere Zugabe von 0,1 bis 10 ml des Alkohols pro 1 g des Präzipitats. Beispielsweise können 0,1 ml, 1 ml, 2 ml, 3 ml, 4 ml, 5 ml, 6 ml, 7 ml, 8 ml, 9 ml oder 10 ml des Alkohols pro 1 g des Präzipitats zugegeben werden. Gemäß einer weiteren Ausführungsform werden 1 bis 5 ml des Alkohols pro 1 g des Präzipitats zugegeben. Insbesondere werden 1 bis 3 ml des Alkohols pro 1 g des Präzipitats zugegeben. "Gemäß einer Ausführungsform umfasst das Verfahren den Schritt p) Erhitzen der Mischung unter Rühren mit einer Wärmequelle auf eine Temperatur im Bereich von 50 bis 95 °C. Beispielsweise kann die Temperatur 50 °C, 60 °C, 70 °C, 80 °C oder 90 °C betragen. Gemäß einer weiteren Ausführungsform wird die Temperatur im Bereich von 60 bis 90 °C eingestellt. Insbesondere wird die Temperatur im Bereich von 70 bis 80 °C eingestellt. Bevorzugt wird das verdampfende Ethanol rückgeführt.

Gemäß einer Ausführungsform umfasst das Verfahren den Schritt q) Abkühlen auf eine Temperatur im Bereich von 20 bis 40 °C über einen Zeitraum von mindestens 1 Stunde. Beispielsweise kann die Temperatur 20 °C, 25 °C, 30 °C, 35 °C oder 40 °C betragen. Gemäß einer weiteren Ausführungsform wird die Temperatur im Bereich von 25 bis 35 °C eingestellt. Das Abkühlen erfolgt vorzugsweise über einen Zeitraum von mindestens 1,5 Stunden. Besonders bevorzugt erfolgt das Abkühlen über einen Zeitraum von mindestens 2,5 Stunden.

Gemäß einer Ausführungsform umfasst das Verfahren den Schritt r) Abtrennung des Präzipitats. Insbesondere erfolgt die Abtrennung durch Filtration. Gemäß einer optionalen Ausführungsform umfasst das Verfahren den Schritt s) Waschen des Präzipitats mit 0,1 bis 5 ml des Alkohols pro 1 g des Präzipitats. Beispielsweise können 0,1 ml, 1 ml, 1,5 ml, 2 ml, 2,5 ml, 3 ml, 4 ml oder 5 ml des Alkohols pro 1 g des Präzipitats verwendet werden. Gemäß einer weiteren Ausführungsform wird das Präzipitat mit 1 bis 3 ml des Alkohols pro 1 g des Präzipitats gewaschen. Insbesondere wird das Präzipitat mit 1,5 bis 2,5 ml des Alkohols pro 1 g des Präzipitats gewaschen. Gemäß einer Ausführungsform umfasst das Verfahren den Schritt t) Waschen des Präzipitats mit 1,0 bis 8,0 ml eines organischen Lösungsmittels pro 1 g des Präzipitats. Beispielsweise können 1,0 ml, 2,0 ml, 3,0 ml, 4,0 ml, 5,0 ml, 6,0 ml, 7,0 ml oder 8,0 ml des organischen Lösungsmittels pro 1 g des Präzipitats verwendet werden. Gemäß einer weiteren Ausführungsform wird das Präzipitat mit 2,0 bis 6,0 ml des organischen Lösungsmittels pro 1 g des Präzipitats gewaschen. Insbesondere wird das Präzipitat mit 3,5 bis 4,5 ml des organischen Lösungsmittels pro 1 g des Präzipitats gewaschen. Das organische Lösungsmittel ist ausgewählt aus Ketonen, Estern, Alkoholen oder Ethern. Bevorzugt ist das Keton Aceton.

Gemäß einer Ausführungsform umfasst das Verfahren den Schritt u) Trocknung des Feststoffs, wobei die Trocknung einen oder mehrere Trocknungsschritte aufweist. Bevorzugt ist mindestens ein Trocknungsschritt eine Vakuum-Trocknung über einen Zeitraum von 1 bis 5 Tagen. Beispielsweise kann der Zeitraum 1 Tag, 2 Tage, 3 Tage, 4 Tage oder 5 Tage betragen. Gemäß einer weiteren Ausführungsform erfolgt die Vakuum-Trocknung über einen Zeitraum von 1,5 bis 4 Tagen. Die Temperatur während der Vakuum-Trocknung liegt vorzugsweise im Bereich von 50 bis 100 °C. Beispielsweise kann die Temperatur 50 °C, 60 °C, 70 °C, 80 °C, 90 °C oder 100 °C betragen. Gemäß einer weiteren Ausführungsform liegt die Temperatur im Bereich von 65 bis 95 °C. Insbesondere liegt die Temperatur gemäß einer Ausführungsform im Bereich von 75 bis 85 °C.

### Verfahren zur Herstellung von Imipridon-201

Grundsätzlich kann jede Imipridon-201 Base in das erfindungsgemäße Imipridon-201-Dihydrochlorid umgewandelt werden. Besonders gute Ergebnisse werden mit einer möglichst reinen Imipridon-201 Base erzielt. Die Erfinder haben ein Verfahren zur Herstellung von Imipridon-201 entwickelt, dass die Imipridon-201 Base in hoher Reinheit erzeugt.

Dazu wird entsprechend dem bekannten Verfahren 1-Benzyl-4-oxopiperidin-3-carbonsäuremethylester hydrochlorid (Verbindung (1)) mit N-(2-Methylbenzyl)-4,5-dihydro-1H-imidazol-2-amin (Verbindung (2)) in einer leicht exothermen Reaktion zu Imipridon-201 kondensiert. Dies geschieht im erfindungsgemäßen Verfahren in Anwesenheit von Natriummethanolat in Methanol bei Raumtemperatur. Die freie Base wird nach wässriger Aufarbeitung aus Methanol kristallisiert und in reiner Form isoliert. Neben Methanol können auch andere Alkohole und weitere mit Wasser gut mischbare Lösungsmittel verwendet werden.

Folglich betrifft die Erfindung gemäß einem fünften Aspekt ein Verfahren zur Herstellung von Imipridon-201.

Gemäß einer Ausführungsform wird das Verfahren ohne Dioxan durchgeführt. Bevorzugt wird das Verfahren ohne Lösungsmittel durchgeführt, die toxisch, krebserregend und/oder umweltschädlich sind.

Gemäß einer Ausführungsform umfasst das Verfahren den Schritt a) Zugabe von N-(2-Methylbenzyl)-4,5-dihydro-1H-imidazol-2-amin (Verbindung (2)) zu einem alkoholischen Lösungsmittel. Die Konzentration der Verbindung (2) im alkoholischen Lösungsmittel beträgt vorzugsweise 10 Gew-% bis 35 Gew-%. Beispielsweise kann die Konzentration 10 Gew-%, 15 Gew-%, 20 Gew-%, 25 Gew-%, 30 Gew-% oder 35 Gew-% betragen. Gemäß einer weiteren Ausführungsform beträgt die Konzentration 15 Gew-% bis 30 Gew-%. Insbesondere beträgt die Konzentration der Verbindung (2) 20 Gew-% bis 25 Gew-% bezogen auf das alkoholische Lösungsmittel. Das alkoholische Lösungsmittel ist bevorzugt Methanol.

Gemäß einer Ausführungsform umfasst das Verfahren den Schritt b) Zugabe von 1-Benzyl-4-oxopiperidin-3-carbonsäuremethylester hydrochlorid (Verbindung (1)) zu einem alkoholischen Lösungsmittel. Die Konzentration der Verbindung (1) im alkoholischen Lösungsmittel beträgt vorzugsweise 15 Gew-% bis 45 Gew-%. Beispielsweise kann die Konzentration 15 Gew-%, 20 Gew-%, 25 Gew-%, 30 Gew-%, 35 Gew-%, 40 Gew-% oder 45 Gew-% betragen bezogen auf das alkoholische Lösungsmittel. Gemäß einer weiteren Ausführungsform beträgt die Konzentration 20 Gew-% bis 40 Gew-%. Insbesondere beträgt die Konzentration der Verbindung (1) 25 Gew-% bis 35 Gew-% bezogen auf das alkoholische Lösungsmittel.

Gemäß einer Ausführungsform umfasst das Verfahren den Schritt c) Zugabe von Natriummethanolat (NaOMe) zu einem alkoholischen Lösungsmittel. Die Konzentration von NaOMe im alkoholischen Lösungsmittel beträgt vorzugsweise 5 Vol.-% bis 35 Vol.-%. Beispielsweise kann die Konzentration 5 Vol.-%, 10 Vol.-%, 15 Vol.-%, 20 Vol.-%, 25 Vol.-%, 30 Vol.-% oder 35 Vol.-% betragen. Gemäß einer weiteren Ausführungsform beträgt die Konzentration 10 Vol.-% bis 30 Vol.-%. Insbesondere beträgt die Konzentration von NaOMe 15 Vol.-% bis 25 Vol.-% bezogen auf das alkoholische Lösungsmittel.

Bevorzugt wird NaOMe zugetropft. Die Temperatur der Lösung liegt vorzugsweise im Bereich von 20 bis 50 °C. Beispielsweise kann die Temperatur 20 °C, 25 °C, 30 °C, 35 °C, 40 °C, 45 °C oder 50 °C betragen. Gemäß einer weiteren Ausführungsform liegt die Temperatur im Bereich von 30 bis 40 °C. Insbesondere liegt die Temperatur gemäß einer Ausführungsform im Bereich von 33 bis 37 °C.

Gemäß einer Ausführungsform umfasst das Verfahren den Schritt d) Einstellen des pH-Wertes auf einen Wert von mehr als 10. Beispielsweise kann der pH-Wert mehr als 10, mehr als 11 oder mehr als 12 betragen.

Gemäß einer Ausführungsform umfasst das Verfahren den Schritt e) Rühren der Reaktionsmischung über einen Zeitraum von mehr als 5 Stunden. Beispielsweise kann der Zeitraum mehr als 5 Stunden, 10 Stunden, 15 Stunden, 20 Stunden, 25 Stunden oder 30 Stunden betragen. Gemäß einer weiteren Ausführungsform wird die Reaktionsmischung im Bereich von 10 bis 30 Stunden gerührt. Insbesondere wird die Reaktionsmischung gemäß einer Ausführungsform im Bereich von 15 bis 25 Stunden gerührt.

Gemäß einer Ausführungsform umfasst das Verfahren den Schritt f) Einstellen des pH-Wertes auf einen Wert im Bereich von 8,0 bis 10,0. Beispielsweise kann der pH-Wert 8,0, 8,5, 9,0, 9,5 oder 10,0 betragen. Gemäß einer weiteren Ausführungsform wird der pH-Wert im Bereich von 8,5 bis 9,5 eingestellt. Insbesondere beträgt der pH-Wert etwa 9,0.

Bevorzugt wird der pH-Wert durch Zugabe von 10 %-iger HCl eingestellt. Die Zugabe erfolgt vorzugsweise über einen Zeitraum von 60 bis 180 Minuten. Beispielsweise kann der Zeitraum 60 Minuten, 90 Minuten, 120 Minuten, 150 Minuten oder 180 Minuten betragen. Gemäß einer weiteren Ausführungsform erfolgt die Zugabe über einen Zeitraum von 100 bis 140 Minuten.

Gemäß einer Ausführungsform umfasst das Verfahren den Schritt g) Rühren der Reaktionsmischung über einen Zeitraum im Bereich von 6 bis 20 Stunden. Beispielsweise kann der Zeitraum 6 Stunden, 8 Stunden, 10 Stunden, 12 Stunden, 14 Stunden, 16 Stunden, 18 Stunden oder 20 Stunden betragen. Gemäß einer weiteren Ausführungsform wird die Reaktionsmischung über einen Zeitraum im Bereich von 10 bis 14 Stunden gerührt.

Gemäß einer Ausführungsform umfasst das Verfahren den Schritt h) Abtrennen des Reaktionsprodukts, Imipridon-201, vom Lösungsmittel. Insbesondere erfolgt die Abtrennung durch Filtration.

Gemäß einer optionalen Ausführungsform umfasst das Verfahren den Schritt i) Reinigen des Reaktionsprodukts, wobei das Reinigen einen oder mehrere Reinigungsschritte aufweist. Bevorzugt umfasst mindestens ein Reinigungsschritt die Zugabe eines Alkohols, insbesondere Methanol, und die anschließende Abtrennung des Alkohols, insbesondere durch Filtration.

Gemäß einer Ausführungsform umfasst das Verfahren den Schritt j) Trocknung des Reaktionsprodukts, wobei die Trocknung über einen Zeitraum von 1 bis 4 Tagen durchgeführt wird. Beispielsweise kann der Zeitraum 1 Tag, 1,5 Tage, 2 Tage, 2,5 Tage, 3 Tage, 3,5 Tage oder 4 Tage betragen. Gemäß einer weiteren Ausführungsform erfolgt die Trocknung über einen Zeitraum von 1,5 bis 4 Tagen. Die Temperatur während der Vakuum-Trocknung liegt vorzugsweise im Bereich von 30 bis 70 °C. Beispielsweise kann die Temperatur 30 °C, 40 °C, 50 °C, 60 °C oder 70 °C betragen. Gemäß einer weiteren Ausführungsform liegt die Temperatur im Bereich von 40 bis 60 °C. Insbesondere liegt die Temperatur gemäß einer Ausführungsform im Bereich von 45 bis 55 °C.

Weiterhin kann das Verfahren die folgenden Schritte enthalten.

Gemäß einer Ausführungsform umfasst das Verfahren den Schritt k) Zugabe des Reaktionsprodukts Imipridon-201 aus Schritt j) zu einem Alkohol, ausgewählt aus Methanol, Ethanol, Propanol, Isopropanol, n-Butanol. Bevorzugt wird Methanol (MeOH) verwendet. Beispielsweise können 0,5 ml, 1,0 ml, 1,5 ml, 2,0 ml, 2,5 ml, 3,0 ml, 4,0 ml, 5,0 ml, 6,0 ml, 7,0 ml, 8,0 ml, 9,0 ml oder 10 ml des Alkohols pro 1 g des Präzipitats verwendet werden. Gemäß einer weiteren Ausführungsform werden 1,0 bis 6 ml des Alkohols pro 1 g des Präzipitats verwendet. Insbesondere werden 1,5 bis 2,5 ml des Alkohols pro 1 g des Präzipitats verwendet.

Gemäß einer Ausführungsform umfasst das Verfahren den Schritt I) Erhitzen der Mischung unter Rühren auf eine Temperatur im Bereich von 40 bis 80 °C. Beispielsweise kann die Temperatur 40 °C, 50 °C, 60 °C, 70 °C oder 80 °C betragen. Gemäß einer weiteren Ausführungsform wird die Temperatur im Bereich von 50 bis 70 °C eingestellt. Insbesondere wird die Temperatur im Bereich von 60 bis 65 °C eingestellt. Während des Erhitzens wird weiterer Alkohol, vorzugsweise Methanol, zugegeben, bis Imipridon-201 gelöst ist.

Gemäß einer Ausführungsform umfasst das Verfahren den Schritt m) Abtrennung von Resten, vorzugsweise durch Filtration. Gemäß einer Ausführungsform erfolgt die Filtration in einen Kolben mit Rührwerk und Rückflusskühler. Bevorzugt erfolgt die Filtration mit einer Porengröße im Bereich von 10 bis 100 µm. Beispielsweise kann die Porengröße 10 µm, 20 µm, 30 µm, 40 µm, 50 µm, 60 µm, 70 µm, 80 µm, 90 µm oder 100 µm betragen. Gemäß einer weiteren Ausführungsform erfolgt die Filtration mit einer Porengröße im Bereich von 16 bis 40 µm.

Gemäß einer Ausführungsform umfasst das Verfahren den Schritt n) Erhitzen der Lösung unter Rühren zum Siedepunkt.

Gemäß einer Ausführungsform umfasst das Verfahren den Schritt o) Ausfällen von Imipridon-201 durch Abkühlen. Das Abkühlen weist einen oder mehrere Abkühlungsschritte auf. Bevorzugt findet mindestens ein Abkühlungsschritt über einen Zeitraum von 10 bis 24 Stunden statt. Beispielsweise kann der Zeitraum 10 Stunden, 12 Stunden, 14 Stunden, 16 Stunden, 18 Stunden, 20 Stunden, 22 Stunden oder 24 Stunden betragen. Gemäß einer weiteren Ausführungsform erfolgt das Abkühlen über einen Zeitraum von 12 bis 20 Stunden.

Gemäß einer Ausführungsform umfasst das Verfahren den Schritt p) Waschen des Präzipitats mit 0,1 bis 3,0 ml eines Alkohols. Beispielsweise kann die Menge 0,1 ml, 0,2 ml, 0,3 ml, 0,4 ml, 0,5 ml, 0,6 ml, 0,7 ml, 0,8 ml, 0,9 ml, 1,0 ml, 1,5 ml, 2,0 ml, 2,5 ml oder 3,0 ml betragen. Gemäß einer weiteren Ausführungsform wird das Präzipitat mit 0,2 bis 1,0 ml des Alkohols gewaschen. Insbesondere wird das Präzipitat mit 0,5 bis 0,7 ml des Alkohols gewaschen.

Gemäß einer Ausführungsform umfasst das Verfahren den Schritt q) Trocknung des Präzipitats, wobei die Trocknung über einen Zeitraum von mindestens 10 Stunden stattfindet. Beispielsweise kann der Zeitraum 10 Stunden, 12 Stunden, 14 Stunden, 16 Stunden, 18 Stunden oder 20 Stunden betragen. Gemäß einer weiteren Ausführungsform erfolgt die Trocknung über einen Zeitraum von mindestens 14 Stunden. Insbesondere erfolgt die Trocknung über einen Zeitraum von mindestens 16 Stunden.

Die Temperatur während der Trocknung liegt vorzugsweise im Bereich von 30 bis 70 °C. Beispielsweise kann die Temperatur 30 °C, 40 °C, 50 °C, 60 °C oder 70 °C betragen. Gemäß einer weiteren Ausführungsform liegt die Temperatur im Bereich von 40 bis 60 °C. Insbesondere liegt die Temperatur gemäß einer Ausführungsform im Bereich von 45 bis 55 °C.

Der Druck während der Trocknung liegt vorzugsweise im Bereich von 10 bis 100 mbar. Beispielsweise kann der Druck 10 mbar, 20 mbar, 30 mbar, 40 mbar, 50 mbar, 60 mbar, 70 mbar, 80 mbar, 90 mbar oder 100 mbar betragen. Gemäß einer weiteren Ausführungsform liegt der Druck im Bereich von 20 bis 40 mbar.

Gemäß einer Ausführungsform des Schritts a) enthält die HCl-haltige wässrige Lösung eine 10 % HCl-Lösung im Bereich von 10 bis 40 Vol.%. Beispielsweise kann die HCl-Lösung 10 Vol.%, 15 Vol.%, 20 Vol.%, 25 Vol.%, 30 Vol.%, 35 Vol.% oder 40 Vol.% betragen. Gemäß einer weiteren Ausführungsform enthält die HCl-Lösung im Bereich von 15 bis 30 Vol.%. Gemäß einer zusätzlichen Ausführungsform enthält die HCl-Lösung im Bereich von 20 bis 25 Vol.%.

Die Umwandlung der freien Base in das Dihydrochlorid-Salz erfolgt in wässriger Salzsäure. Das reine Salz wird durch Aceton ausgefällt und anschließend in Ethanol 96% umkristallisiert. Neben Aceton können auch andere organische Lösungsmittel wie z.B. solche aus den Klassen der Ketone, Ester, Alkohole oder Ether, insbesondere diejenigen der pharmazeutisch akzeptablen Lösungsmittel verwendet werden.

Das Verfahrensprodukt dieses Verfahrens, die Base Imipridon-201 ist besonders rein. Gemäß einer Ausführungsform weist die Imipridon-201 Base eine ICH-konforme Reinheit auf, wobei - gemessen durch UV-Spektroskopie - bei Wellenlängen oberhalb 500 nm keine Verunreinigungen nachgewiesen werden können.

### Verfahren zur Herstellung von Imipridon-206

Grundsätzlich kann jede Imipridon-201 Base in das erfindungsgemäße Imipridon-201-Dihydrochlorid umgewandelt werden. Besonders gute Ergebnisse werden mit einer möglichst reinen Imipridon-201 Base erzielt. Die Erfinder haben ein Verfahren zur Herstellung von Imipridon-201 entwickelt, dass die Imipridon-201 Base in hoher Reinheit erzeugt.

Dazu wird entsprechend dem bekannten Verfahren 1-Benzyl-4-oxopiperidin-3-carbonsäuremethylester hydrochlorid (1) mit N-(2,4-Difluorbenzyl)-4,5-dihydro-1H-imidazol-2-amin (Verbindung (3)) in einer leicht exothermen Reaktion zu Imipridon-206 kondensiert. Dies geschieht im erfindungsgemäßen Verfahren in Anwesenheit von Natriummethanolat in Methanol bei Raumtemperatur. Die freie Base wird nach wässriger Aufarbeitung aus Ethanol 96% kristallisiert und in reiner Form isoliert. Neben Ethanol können auch andere Alkohole und weitere mit Wasser gut mischbare Lösungsmittel verwendet werden.

Folglich betrifft die Erfindung gemäß einem sechsten Aspekt ein Verfahren zur Herstellung von Imipridon-206.

Gemäß einer Ausführungsform wird das Verfahren ohne Dioxan durchgeführt. Bevorzugt wird das Verfahren ohne Lösungsmittel durchgeführt, die toxisch, krebserregend und/oder umweltschädlich sind.

Gemäß einer Ausführungsform umfasst das Verfahren den Schritt a) Zugabe von N-(2-Methylbenzyl, 4-Difluorbenzyl)-4,5-dihydro-1H-imidazol-2-amin (Verbindung (3)) zu einem alkoholischen Lösungsmittel. Die Konzentration der Verbindung (3) im alkoholischen Lösungsmittel beträgt vorzugsweise 15 bis 35 Gew.-%. Beispielsweise kann die Konzentration 15 Gew.-%, 20 Gew.-%, 25 Gew.-%, 30 Gew.-% oder 35 Gew.-% betragen. Gemäß einer weiteren Ausführungsform beträgt die Konzentration 20 bis 30 Gew.-%. Insbesondere beträgt die Konzentration 22 bis 28 Gew.-% bezogen auf das alkoholische Lösungsmittel. Das alkoholische Lösungsmittel ist bevorzugt Methanol.

Gemäß einer Ausführungsform umfasst das Verfahren den Schritt b) Zugabe von 1-Benzyl-4-oxopiperidin-3-carbonsäuremethylester hydrochlorid (Verbindung (1)) zu einem alkoholischen Lösungsmittel. Die Konzentration der Verbindung (1) im alkoholischen Lösungsmittel beträgt vorzugsweise 20 bis 40 Gew.-%. Beispielsweise kann die Konzentration 20 Gew.-%, 25 Gew.-%, 30 Gew.-%, 35 Gew.-% oder 40 Gew.-% betragen. Gemäß einer weiteren Ausführungsform beträgt die Konzentration 25 bis 35 Gew.-%. Insbesondere beträgt die Konzentration 28 bis 33 Gew.-% bezogen auf das alkoholische Lösungsmittel.

Gemäß einer Ausführungsform umfasst das Verfahren den Schritt c) Zugabe von Natriummethanolat (NaOMe) zu einem alkoholischen Lösungsmittel. Die Konzentration von NaOMe im alkoholischen Lösungsmittel beträgt vorzugsweise 10 Vol.-% bis 40 Vol.-%. Beispielsweise kann die Konzentration 10 Vol.-%, 15 Vol.-%, 20 Vol.-%, 25 Vol.-%, 30 Vol.-%, 35 Vol.-% oder 40 Vol.-% betragen. Gemäß einer weiteren Ausführungsform beträgt die Konzentration 20 Vol.-% bis 30 Vol.-%. Insbesondere beträgt die Konzentration 23 Vol.-% bis 27 Vol.-% bezogen auf das alkoholische Lösungsmittel. Bevorzugt wird NaOMe zugetropft. Die Temperatur der Lösung liegt vorzugsweise im Bereich von 20 bis 50 °C. Beispielsweise kann die Temperatur 20 °C, 25 °C, 30 °C, 35 °C, 40 °C, 45 °C oder 50 °C betragen. Gemäß einer weiteren Ausführungsform liegt die Temperatur im Bereich von 30 bis 40 °C. Insbesondere liegt die Temperatur gemäß einer Ausführungsform im Bereich von 33 bis 37 °C.

Gemäß einer Ausführungsform umfasst das Verfahren den Schritt d) Einstellen des pH-Wertes auf einen Wert von mehr als 10. Beispielsweise kann der pH-Wert mehr als 10, mehr als 11 oder mehr als 12 betragen."

Gemäß einer Ausführungsform umfasst das Verfahren den Schritt e) Rühren der Reaktionsmischung über einen Zeitraum von mehr als 5 Stunden. Beispielsweise kann der Zeitraum mehr als 5 Stunden, 10 Stunden, 15 Stunden, 20 Stunden, 25 Stunden oder 30 Stunden betragen. Gemäß einer weiteren Ausführungsform wird die Reaktionsmischung im Bereich von 10 bis 30 Stunden gerührt. Insbesondere wird die Reaktionsmischung gemäß einer Ausführungsform im Bereich von 15 bis 25 Stunden gerührt.

Gemäß einer Ausführungsform umfasst das Verfahren den Schritt f) Einstellen des pH-Wertes auf einen Wert im Bereich von 8,0 bis 10,0. Beispielsweise kann der pH-Wert 8,0, 8,5, 9,0, 9,5 oder 10,0 betragen. Gemäß einer weiteren Ausführungsform wird der pH-Wert im Bereich von 8,5 bis 9,5 eingestellt. Insbesondere beträgt der pH-Wert etwa 9,0. Bevorzugt wird der pH-Wert durch Zugabe von 10 %-iger HCl eingestellt. Die Zugabe erfolgt vorzugsweise über einen Zeitraum von 60 bis 180 Minuten. Beispielsweise kann der Zeitraum 60 Minuten, 90 Minuten, 120 Minuten, 150 Minuten oder 180 Minuten betragen. Gemäß einer weiteren Ausführungsform erfolgt die Zugabe über einen Zeitraum von 100 bis 140 Minuten.

Gemäß einer Ausführungsform umfasst das Verfahren den Schritt g) Rühren der Reaktionsmischung über einen Zeitraum im Bereich von 6 bis 20 Stunden. Beispielsweise kann der Zeitraum 6 Stunden, 8 Stunden, 10 Stunden, 12 Stunden, 14 Stunden, 16 Stunden, 18 Stunden oder 20 Stunden betragen. Gemäß einer weiteren Ausführungsform wird die Reaktionsmischung über einen Zeitraum im Bereich von 10 bis 14 Stunden gerührt.

Gemäß einer Ausführungsform umfasst das Verfahren den Schritt h) Abtrennen des Reaktionsprodukts, Imipridon-206, vom Lösungsmittel. Insbesondere erfolgt die Abtrennung durch Filtration."

Gemäß einer optionalen Ausführungsform umfasst das Verfahren den Schritt i) Reinigen des Reaktionsprodukts, wobei das Reinigen einen oder mehrere Reinigungsschritte aufweist. Bevorzugt umfasst mindestens ein Reinigungsschritt die Zugabe eines Alkohols, insbesondere Methanol, und die anschließende Abtrennung des Alkohols, insbesondere durch Filtration.

Gemäß einer Ausführungsform umfasst das Verfahren den Schritt j) Trocknung des Reaktionsprodukts, wobei die Trocknung über einen Zeitraum von 1 bis 4 Tagen durchgeführt wird. Beispielsweise kann der Zeitraum 1 Tag, 1,5 Tage, 2 Tage, 2,5 Tage, 3 Tage, 3,5 Tage oder 4 Tage betragen. Gemäß einer weiteren Ausführungsform erfolgt die Trocknung über einen Zeitraum von 1,5 bis 4 Tagen. Die Temperatur während der Vakuum-Trocknung liegt vorzugsweise im Bereich von 30 bis 70 °C. Beispielsweise kann die Temperatur 30 °C, 40 °C, 50 °C, 60 °C oder 70 °C betragen. Gemäß einer weiteren Ausführungsform liegt die Temperatur im Bereich von 40 bis 60 °C. Insbesondere liegt die Temperatur gemäß einer Ausführungsform im Bereich von 45 bis 55 °C.

Die überraschend hohe Reinheit und exzellente Langzeitstabilität werden offenbar durch das erfindungsgemäße Vorgehen erzeugt.

Es ist davon auszugehen, dass die Wirkstoffe durch die erfindungsgemäßen Herstellungsverfahren "besonders gut" auskristallisieren. So erfolgt im erfindungsgemäßen Verfahren eine basenkatalysierte Synthese der freien Base, wohingegen im Stand der Technik auch eine säurekatalysierte Synthese unter Einsatz von PPTS (Pyridinium-p-Toluolsulfonat) bekannt ist. Das erfindungsgemäße Verfahren verzichtet auf diese Chemikalie. Im Unterschied zum Stand der Technik wurde bei basenkatalysierter Reaktionsführung beobachtet, dass eine Reaktionsführung bei erhöhter Temperatur, insbes. bei Rückfluss nicht notwendig ist und die Reaktion bei Raumtemperatur durchgeführt werden kann, wobei sich durch eine leichte Exothermie bei Zugabe von Natriummethanolat die Mischung etwas erwärmen kann. Der Überschuss an einem Ausgangsmaterial, Verbindung (2) kann zudem stark reduziert und durch Natriummethanolat als einfaches Reagenz ersetzt werden. Das erfindungsgemäße Verfahren verzichtet auf eine extraktive Aufarbeitung und chromatographische Aufreinigung mit halogenhaltigen Lösungsmitteln, die zudem mehrfach zur Trockne eingeengt werden und liefert das Produkt als Kristallisat, das noch mit einfachen Mitteln umkristallisiert werden kann, um die sehr hohe Reinheit der nachfolgenden Salzbildung zu gewährleisten, bei der keine Verunreinigung größer 0,1% enthalten ist. Somit ist das erfindungsgemäße Verfahren durch eine energieeffiziente und ressourcenschonende Vorgehensweise gekennzeichnet.

Im Stand der Technik wird aus der Reaktionsmischung der freien Base das Dihydrochlorid ausgefällt. Bereits in diesem ersten Schritt der Salzbildung unterscheidet sich die erfindungsgemäße Vorgehensweise, bei der die freie Base vor der Weiterverarbeitung in Reinform isoliert und umkristallisiert wird.

Im zweiten Schritt erfolgt die Fällung des Salzes nach dem Stand der Technik mit Chlorwasserstoff in organischer Phase mit hochtoxischem 1,4-Dioxan. Dioxan gehört nachweislich zu den besonders krebserregenden Stoffen (CMR-Stoffe, d.h. cancerogen, mutagen und reproduktionstoxisch) mit schwerwiegenden Auswirkungen nicht nur auf die menschliche Gesundheit, sondern auch auf die Umwelt. Chlorwasserstoff, gelöst in organischen Lösungsmitteln ist zudem stark korrosiv und erfordert zusätzlich besondere Aufmerksamkeit im sicheren Umgang.

Im Gegensatz dazu besteht der wesentliche Unterschied des erfindungsgemäßen Verfahrens darin, dass auf krebserregende Lösungsmittel vollständig verzichtet werden kann und vor allem dioxanfrei gearbeitet werden kann. Weiter besteht ein Unterschied darin, dass die Bildung des Salzes mit wässriger Säure erfolgt. Die Ausfällung aus dieser wässrigen Lösung gelingt mit Hilfe eines unkritischen organischen Lösungsmittels. Auf aufwändige Destillationsschritte und Azeotropierungen kann hier verzichtet werden. Somit ist das erfindungsgemäße Verfahren durch eine energieeffiziente und ressourcenschonende Vorgehensweise gekennzeichnet.

Ein weiterer Vorteil des erfindungsgemäßen Verfahrens besteht darin, dass auf Inertbegasung vollständig verzichtet werden kann.

Nach dem Stand der Technik ist ein erhöhter Personenschutz aufgrund der Verwendung von Dioxan zwingend erforderlich, der beim erfindungsgemäßen Verfahren nicht notwendig ist. Das bevorzugt in wässriger Phase aufgereinigte Dihydrochlorid bildet offensichtlich Kristalle aus, die keine oder nur minimale amorphe Bestandteile enthalten. Die Ausfällung erfolgt vollständig und ist technologisch gut zu beherrschen. Dies steht im Ggs. zum Std. d. T., nach dem die Auskristallisation schwierig und nur unter genauer Einhaltung der Parameter und Reaktionsbedingungen möglich ist.

Durch die erfindungsgemäße Herstellung lässt sich die Filtration leicht und effektiv durchführen. Durch die sehr gute Kristallbildung ist auch die Ausbeute gegenüber dem Std. d. T. deutlich erhöht. So erhält man nach Verfahren des Standes der Technik eine Ausbeute von lediglich 49%, während das erfindungsgemäße Verfahren durchweg Ausbeuten ≥ 60% bei einer deutlich höheren Reinheit (% area HPLC) auf dieser Verfahrensstufe liefert. Durch die erfindungsgemäße Aufarbeitung und Kristallisation lassen sich die Verluste in der Gesamtausbeute auf ein Minimum reduzieren. So beträgt die Gesamtausbeute mindestens ≥60%, bevorzugt mindestens ≥80% und besonders bevorzugt mindestens ≥90%, wobei eine Steigerung der Gesamtausbeute durch weitere Umkristallisationen erreicht und nahezu verlustfrei gearbeitet wird.

Durch die erfindungsgemäße Verfahrensweise wird somit ein hocheffizientes, wirtschaftliches und umweltschonendes Verfahren vorgeschlagen unter Vermeidung hochtoxischer Lösungsmittel und unter Verzicht auf zusätzliche Chemikalien wie bisher aus dem Std. d. T. bekannt.

### Pharmazeutische Zusammensetzung

Gemäß einem siebten Aspekt betrifft die Erfindung eine pharmazeutische Zusammensetzung zur Verwendung in der Behandlung von Krebs umfassend ein Dihydrochlorid-Salz von Imipridon-201 gemäß dem ersten Aspekt und/oder ein Dihydrochlorid-Salz von Imipridon-206 gemäß dem zweiten Aspekt. Die Zusammensetzung enthält zudem einen pharmazeutisch akzeptablen Hilfsstoff und/oder einen pharmazeutisch akzeptablen Träger.

Gemäß einer Ausführungsform enthält die Pharmazeutische Zusammensetzung die röntgenographische Kristallform von Imipridon-201 in einer Gesamtmenge von mindestens 5 Gew.-%, wobei diese Gesamtmenge vorzugsweise mindestens 10, 15, 20, 25, 30, 35, 40, 45, 50, 55, 60, 65, 70, 75, 80, 85, 90, 95 Gew.-% oder mehr, beträgt, jeweils bezogen auf die in der Zusammensetzung enthaltene Gesamtmenge an Wirkstoff. Unter "**Gesamtmenge**" wird in der vorliegenden Erfindung die therapeutisch notwendige Dosierung verstanden.

Die pharmazeutische Zusammensetzung kann in jeder üblichen pharmazeutischen Darreichungsform vorliegen für die Applikationswege oral, bukkal, peroral, nasal, inhalativ, parenteral, subkutan etc. Beispiele für Arzneiformen sind Tabletten, Kapseln, Pulver, Granulate.

Pharmazeutisch akzeptable Hilfsstoffe und Träger sind Substanzen, die in pharmazeutischen Formulierungen verwendet werden, um die Stabilität, Bioverfügbarkeit, und Verabreichungsform eines Medikaments zu verbessern, ohne selbst pharmakologisch aktiv zu sein. Pharmazeutisch akzeptable Hilfsstoffe sind beispielsweise Bindemittel, wie Mikrokristalline Cellulose, Polyvinylpyrrolidon (PVP), oder Hydroxypropylmethylcellulose (HPMC); Füllstoffe, wie Lactose, Mannitol, oder Dicalciumphosphat; Gleitmittel, wie Magnesiumstearat, Stearinsäure, oder Talkum; Feuchthaltemittel, wie Glycerin, Sorbitol, oder Propylenglykol, Konservierungsmittel, wie Benzalkoniumchlorid, Parabene (Methylparaben, Propylparaben) oder Phenol; Stabilisatoren wie EDTA (Ethylendiamintetraessigsäure), Zitronensäure, oder Ascorbinsäure, Farbstoffe wie Titandioxid, Eisenoxide, FD&C Farben, Geschmacksstoffe, wie Saccharose, Aspartam, oder Acesulfam-K; Süßstoffe, wie Saccharin, Sucralose, oder Stevia; Disintegrationsmittel, Stärkederivate (z.B. Natriumstärkeglykolat), Crospovidon, oder Croscarmellosenatrium

Pharmazeutisch akzeptable Träger sind beispielsweise Lösungsmittel, wie Wasser für Injektionszwecke, Ethanol, oder Propylenglykol; Öle, wie Pflanzenöle (z.B. Sojaöl, Olivenöl), oder Mittelkettige Triglyceride (MCT); Fette, wie Kakaobutter, Lanolin, oder Hartfett, Basen für Zäpfchen, wie Witepsol, Suppocire, Glyceride, Emulgatoren, Polysorbat 80, Lecithin, Sorbitanfettsäureester; Gele, wie Carbopol, Gelatine, oder Agar; Polymere für kontrollierte Freisetzung wie Ethylcellulose, Polylactid-co-Glycolid (PLGA), Eudragit.

### BEISPIELE

### Beispiel 1 - Herstellung von Imipridon-201

69,4 g N-(2-Methylbenzyl)-4,5-dihydro-1H-imidazol-2-amin (Verbindung (2), 0,367 mol) werden in 300 ml Methanol gelöst. Zur leicht gelblichen Lösung werden 94,6 g 1-Benzyl-4-oxopiperidin-3-carbonsäuremethylester hydrochlorid (Verbindung (1), 0,333 mol) zugegeben, wobei sich eine leicht trübe, rotbraune Lösung bildet. Bei Raumtemperatur werden insgesamt 68 ml Natriummethanolat (5,4 M in Methanol, 0,367 mol) bei max. 35°C zugetropft (leicht exotherm) und die Mischung über Nacht ausgerührt. Anschließend werden 30 ml Salzsäure (10%) bis pH 8,2 bis 8,3 über 90 min, gefolgt von 220 ml Wasser über 8 Stunden zugegeben. Nach weiteren 12 Stunden wird die Suspension abfiltriert, der Rückstand mit 125 ml Methanol/Wasser 3:2 kalt gewaschen und gut trockengesaugt. Das Rohprodukt wird in 320 ml Methanol/Wasser 1:9 suspendiert und 2 Stunden gerührt, davon 20 Minuten mit Anwendung von Ultraschall. Der Feststoff wird abfiltriert, mit 2x 125 ml Methanol/Wasser 1:9 gewaschen und bei 50°C im Vakuum getrocknet. Ausbeute 110 g (0,285 mol, 85%) leicht gelbliches Pulver.

Durch Umkristallisation aus bis zu 300 ml Methanol wird nach Heißfiltration, Abkühlen im Eisbad, Filtration, Waschen mit 65 ml kaltem Methanol und Trocknen bei 50°C im Vakuum ein weißes Pulver erhalten. Gesamtausbeute 90 g (0,233 mol, 70%). HPLC Reinheit 100%.

### Beispiel 2 - Herstellung von Imipridon-201-dihydrochlorid

Zu 72 ml Wasser werden bei 60°C unter Rühren abwechselnd und in Portionen insgesamt 50 g Imipridon-201 (0,13 mol) und ca. 77 ml Salzsäure (10%) zugegeben, bis der pH-Wert etwa 3,5 beträgt und eine fast klare Lösung entstanden ist. Diese wird filtriert und bei 50°C werden unter Rühren weitere ca. 20 ml Salzsäure (10%) langsam zugetropft, bis der pH 0,8 beträgt. Zur Lösung werden zügig 500 ml Aceton zugetropft. Beim Abkühlen auf Raumtemperatur setzt die Kristallisation ein. Zu dieser Suspension werden über mehrere Stunden weitere 1,86 Liter Aceton zugetropft. Nach einer weiteren Stunde nachrühren wird der Feststoff im Vakuum abfiltriert, zweimal mit je 200 ml Aceton gewaschen, trockengesaugt und im Vakuum bei 50°C für mindestens 16 Stunden getrocknet. Das Rohprodukt wird aus bis zu 850 ml Ethanol 96% umkristallisiert, wobei die entstehende Suspension bei Raumtemperatur mindestens 10 Stunden nachgerührt wird. Nach Filtration wird der Feststoff mit 100 ml kaltem Ethanol 96% und 200 ml Aceton gewaschen und trockengesaugt. Das Produkt wird bei 80°C im Vakuum für mindestens 16 Stunden getrocknet. Nach Siebung wird das Produkt im Vakuum bei 80°C fertig getrocknet. Ausbeute: ca. 45 g weißes Pulver (ca. 0,10 mol, 75%). HPLC Reinheit 100%.

### Beispiel 3 - Herstellung von Imipridon-206

7,0 g N-(2,4-Difluorbenzyl)-4,5-dihydro-1H-imidazol-2-amin (Verbindung (3), 33,0 mmol) werden in 27 ml Methanol gelöst. Zur leicht gelblichen Lösung werden 8,5 g 1-Benzyl-4-oxopiperidin-3-carbonsäuremethylester hydrochlorid (Verbindung (1), 30,0 mmol) zugegeben, wobei sich eine leicht trübe, rotbraune Lösung bildet. Bei Raumtemperatur werden insgesamt 6,9 ml Natriummethanolat (5,3 M in Methanol, 36,5 mmol) zugetropft (leicht exotherm) und die Mischung über Nacht ausgerührt. Anschließend werden 3,8 ml Salzsäure (10%) bis pH 8,5, gefolgt von 18 ml Wasser über 4 Stunden zugegeben. Am nächsten Tag wird die Suspension abfiltriert und der Rückstand mit wenig Methanol/Wasser 3:2 kalt gewaschen. Der Feststoff wird bei 50°C im Vakuum getrocknet. Ausbeute 10,9 g (26,7 mmol, 89%) leicht beiges Pulver.

Durch Umkristallisation aus 130 ml Ethanol 96% wird nach Abkühlen im Eisbad, Filtration, Waschen mit wenig kaltem Ethanol 96% und Trocknen bei 50°C im Vakuum ein leicht gelbes Pulver erhalten. Gesamtausbeute 8,5 g (20,8 mmol, 70%). HPLC Reinheit 99,9%.

### Beispiel 4 - Herstellung von Imipridon-206-dihydrochlorid

Zu 70 ml Wasser werden bei 60°C unter Rühren abwechselnd und in Portionen insgesamt 50 g Imipridon-206 (0,12 mol) und ca. 76 ml Salzsäure (10%) zugegeben, bis der pH-Wert etwa 4,2 beträgt und eine fast klare Lösung entstanden ist. Diese wird filtriert und bei 50°C werden unter Rühren weitere ca. 19 ml Salzsäure (10%) langsam zugetropft, bis der pH 1,1 beträgt. Zur Lösung werden zügig 480 ml Aceton zugetropft. Beim Abkühlen auf Raumtemperatur setzt die Kristallisation ein. Zu dieser Suspension werden über mind. 4 Stunden weitere 1,75 Liter Aceton zugetropft. Nach mindestens einer weiteren Stunde nachrühren wird der Feststoff im Vakuum abfiltriert, zweimal mit je 200 ml Aceton gewaschen und trockengesaugt. Das Rohprodukt wird aus bis zu 300 ml Ethanol 96% umkristallisiert, wobei die entstehende Suspension bei Raumtemperatur mindestens 16 Stunden nachgerührt wird. Nach Filtration wird der Feststoff mit 50 ml kaltem Ethanol 96% und 200 ml Aceton gewaschen und trockengesaugt. Das Produkt wird bei 80°C im Vakuum für mindestens 16 Stunden getrocknet. Nach Siebung wird das Produkt im Vakuum bei 80°C fertig getrocknet. Ausbeute: ca. 49 g weißes Pulver (ca. 0,10 mol, 83%). HPLC Reinheit 100%.

### Beispiel 5 - Bestimmung des Röntgenpulverdiffraktogramme

### 5.1 Versuchsdurchführung

Zur Charakterisierung des Kristallformen der Salze wurde die Röntgenpulverdiffraktometrie wie folgt durchgeführt:

### Trocknen der Probe:

Der Imipridon-201-dihydrochlorid wird getrocknet, um jegliche Feuchtigkeit zu entfernen, die die XRD-Messung beeinträchtigen könnte.

### Mahlen der Probe:

Das getrocknete Salz wird zu einem feinen Pulver gemahlen. Dies ist wichtig, um sicherzustellen, dass die Kristalle in zufälligen Orientierungen vorliegen, was eine gleichmäßige Beugung der Röntgenstrahlen gewährleistet. Das Mahlen kann manuell mit einem Mörser und Stößel oder maschinell mit einem Kugelmühlen erfolgen.

### Sieben der Probe:

Das gemahlene Pulver wird durch ein feines Sieb (z.B. 100-200 Mesh) gesiebt, um gleichmäßig feine Partikel zu erhalten und grobe Agglomerate zu entfernen.

### Befüllen der Probenhalterung:

Eine kleine Menge des feinen Pulvers wird in eine Probenhalterung (oft eine flache Vertiefung oder ein Probenhalter aus Aluminium oder Kunststoff) gegeben.

Das Pulver sollte gleichmäßig und ohne Hohlräume verteilt werden. Manchmal wird eine Glasplatte oder ein Spatel verwendet, um die Oberfläche zu glätten.

### Einsetzen der Probe in das Diffraktometer:

Die Probenhalterung wird in das Röntgenpulverdiffraktometer eingesetzt. Die Position der Probe muss genau justiert werden, um eine korrekte Messung zu gewährleisten.

### Einstellung der Messparameter:

Automatische Justierung auf den Nullpunkt der Goniometerachse des Diffraktometers

| | |
|---|---|
| Geometrie: | Bragg-Brentano, Reflexionsmodus bzw. |
| | PANalytical Empyrean Diffraktometer (Transmission)* |
| Winkelbereich: | [°2Theta] = 2 - 50 |
| Anodenmaterial: | Cu |
| Wellenlänge: | Cu-K-α, λ = 1.5418 Å (Imipridon-201 -dihydrochlorid) |
| | λ = 1,54056 Å (Imipridon-206-dihydrochlorid) |
| El. Parameter: | U = 40kV, I = 7.5mA |
| Angabe 2Θ (2 Theta)-Werte: | ± 0,2° |

### Messung:

Das Diffraktometer bestrahlt die Probe mit Röntgenstrahlen und misst die Intensität der gebeugten Strahlen bei verschiedenen 2θ-Winkeln.

Ein Detektor erfasst die Intensität der gebeugten Strahlen und erstellt ein Diffraktogramm, das die Intensität der Strahlen als Funktion des 2θ-Winkels zeigt.

### Datensammlung:

Die gesammelten Daten werden in Form eines Diffraktogramms gespeichert, das Peaks bei bestimmten 2θ-Werten zeigt, die charakteristisch für die Kristallstruktur des Wirkstoffsalzes sind.

### 5.2 Erfindungsgemäßes röntgenographisches Imipridon-201-dihydrochlorid

Das Ergebnis der Messung von dem erfindungsgemäße röntgenographischen Imipridon-201-dihydrochlorid ist in **Figur 1** dargestellt.

In der folgenden **Tabelle 1** sind sämtliche Peaks des Diffraktogramms dargestellt.

**Tabelle 2. Peaks aus dem Diffraktogramm des erfindungsgemäßen Imipridon-201-dihydrochlorid**

| | 2Θ (2Theta)-Werte in ° | Intensität (relativ) | Intensität (absolut) |
|---|---|---|---|
| (1) | 4,8 | 9,93 | 2565 |
| (2) | 6,9 | 29,9 | 7723 |
| (3) | 7,7 | 68,74 | 17755 |
| (4) | 8,2 | 1,55 | 399 |
| (5) | 9,6 | 100 | 25831 |
| (6) | 11,3 | 13,37 | 3454 |
| (7) | 12,0 | 2,71 | 701 |
| (8) | 12,9 | 19,66 | 5079 |
| (9) | 13,9 | 10,62 | 2744 |
| (10) | 14,3 | 15,85 | 4095 |
| (11) | 14,7 | 17,48 | 4516 |
| (12) | 15,1 | 4,6 | 1189 |
| (13) | 15,5 | 27,74 | 7166 |
| (14) | 16,0 | 3,44 | 889 |
| (15) | 16,2 | 2,48 | 641 |
| (16) | 16,4 | 3,74 | 967 |
| (17) | 16,7 | 2,14 | 553 |
| (18) | 17,1 | 4,21 | 1088 |
| (19) | 17,7 | 5,81 | 1500 |
| (20) | 18,1 | 7,58 | 1957 |
| (21) | 18,3 | 8,12 | 2097 |
| (22) | 18,5 | 3,65 | 943 |
| (23) | 18,7 | 2,9 | 749 |
| (24) | 19,0 | 2,11 | 545 |
| (25) | 19,4 | 4,81 | 1242 |
| (26) | 19,7 | 3,65 | 943 |
| (27) | 20,3 | 11,81 | 3050 |
| (28) | 20,7 | 5,2 | 1343 |
| (29) | 21,2 | 23,86 | 6162 |
| (30) | 21,4 | 58,73 | 15170 |
| (31) | 22,0 | 2,24 | 579 |
| (32) | 22,5 | 24,18 | 6246 |
| (33) | 23,1 | 27,32 | 7058 |
| (34) | 23,6 | 6,99 | 1805 |
| (35) | 24,1 | 5,93 | 1532 |
| (36) | 24,4 | 5,39 | 1392 |
| (37) | 24,9 | 3,9 | 1007 |
| (38) | 25,3 | 27,96 | 7221 |
| (39) | 25,7 | 10,19 | 2631 |
| (40) | 25,9 | 7,6 | 1964 |
| (41) | 26,9 | 19,88 | 5135 |
| (42) | 27,7 | 10,95 | 2829 |
| (43) | 28,0 | 8,01 | 2068 |
| (44) | 28,8 | 1,55 | 400 |
| (45) | 29,2 | 4,01 | 1035 |
| (46) | 29,7 | 1,08 | 278 |
| (47) | 31,0 | 3,62 | 936 |
| (48) | 31,3 | 8,72 | 2252 |
| (49) | 31,9 | 2,93 | 757 |
| (50) | 32,5 | 5,55 | 1434 |
| (51) | 33,7 | 7,66 | 1980 |
| (52) | 34,4 | 1,73 | 446 |
| (53) | 34,7 | 2,55 | 658 |
| (54) | 35,5 | 1,3 | 335 |
| (55) | 36,5 | 3,92 | 1012 |
| (56) | 37,9 | 3,02 | 780 |
| (57) | 38,3 | 2,06 | 532 |
| (58) | 38,6 | 2,28 | 588 |
| (59) | 39,3 | 3,14 | 810 |
| (60) | 39,9 | 0,22 | 56 |

Der Peak mit der höchsten Intensität ist der Peak (5) bei 9,2°. Dessen Intensität (25831) wurde als Höchstwert auf 100 % gesetzt. Entsprechend wurde den anderen Peaks ein relativer (prozentualer) Wert bezogen auf die maximale Intensität zugeordnet. Die Peaks (2), (3), (5), (8), (13), (29), (30), (32), (33), (38), (41) weisen eine relative Intensität von mindestens 20 % auf, so dass diese Peaks auch dann gut erkennbar sein sollten wenn partiell andere Kristallformen vorkommen.

### 5.3 Imipridon-201-dihydrochlorid gemäß Stand der Technik

Imipridon-201-dihydrochlorid wurde zum Einen von der Firma MedKoo Biosciences Cat 206992, Inc. erworben. Zum anderen wurde Imipridon-201-dihydrochlorid nach einem im Stand der Technik beschriebenen Verfahren hergestellt (EP 2698294 Bsp. 1 und 2)

Das Ergebnis der Messung von dem nachgearbeiteten Imipridon-201-dihydrochlorid ist in **Figur 2** dargestellt. Das Refraktogramm des Imipridon-201-dihydrochlorid Produkts von MedKoo Biosciences, Inc. wies dieselben Peaks auf.

### 5.4 Erfindungsgemäßes Imipridon-206-dihydrochlorid

Das Ergebnis der Messung von dem erfindungsgemäße röntgenographischen Imipridon-206-dihydrochlorid ist in **Figur 3** dargestellt.

In der folgenden **Tabelle 2** sind sämtliche Peaks des Diffraktogramms dargestellt.

**Tabelle 2: Peaks aus dem Diffraktogramm des erfdindungsgemäßen Imipridon-206-dihydrochlorid**

| | 2Θ (2Theta)-Werte in ° | Intensität (relativ) | Intensität (absolut) |
|---|---|---|---|
| (1) | 6,0 | 5.92 | 6549 |
| (2) | 6,5 | 17.58 | 19437 |
| (3) | 6,9 | 5.09 | 5627 |
| (4) | 7,7 | 4.79 | 5293 |
| (5) | 9,0 | 4.63 | 5115 |
| (6) | 10,0 | 11.80 | 13049 |
| (7) | 11,5 | 4.55 | 5028 |
| (8) | 12,0 | 3.12 | 3448 |
| (9) | 13,0 | 4.51 | 4991 |
| (10) | 13,5 | 14.57 | 16108 |
| (11) | 13,6 | 12.18 | 13466 |
| (12) | 14,2 | 100.00 | 110562 |
| (13) | 14,8 | 3.68 | 4072 |
| (14) | 15,6 | 16.09 | 17795 |
| (15) | 16,1 | 15.69 | 17349 |
| (16) | 16,6 | 12.68 | 14025 |
| (17) | 16,9 | 4.01 | 4437 |
| (18) | 18,1 | 10.16 | 11238 |
| (19) | 18,9 | 7.71 | 8523 |
| (20) | 19,0 | 6.85 | 7572 |
| (21) | 19,6 | 3.98 | 4397 |
| (22) | 19,9 | 6.39 | 7068 |
| (23) | 20,1 | 5.49 | 6065 |
| (24) | 20,4 | 10.89 | 12037 |
| (25) | 21,3 | 40.04 | 44274 |
| (26) | 21,8 | 4.63 | 5114 |
| (27) | 22,1 | 4.45 | 4925 |
| (28) | 22,5 | 5.36 | 5924 |
| (29) | 22,9 | 6.93 | 7664 |
| (30) | 23,7 | 7.15 | 7907 |
| (31) | 23,8 | 6.80 | 7523 |
| (32) | 24,9 | 8.10 | 8956 |
| (33) | 25,5 | 68.88 | 76155 |
| (34) | 26,0 | 52.20 | 57709 |
| (35) | 26,3 | 18.71 | 20684 |
| (36) | 27,1 | 6.53 | 7215 |
| (37) | 27,4 | 17.23 | 19050 |
| (38) | 27,7 | 43.53 | 48122 |
| (39) | 28,2 | 11.23 | 12416 |
| (40) | 28,7 | 8.59 | 9502 |
| (41) | 29,1 | 15.24 | 16845 |
| (42) | 29,6 | 4.03 | 4451 |
| (43) | 30,2 | 3.66 | 4049 |
| (44) | 30,4 | 4.76 | 5258 |
| (45) | 30,7 | 4.73 | 5225 |
| (46) | 31,0 | 5.83 | 6441 |
| (47) | 31,4 | 2.73 | 3016 |
| (48) | 32,1 | 5.15 | 5690 |
| (49) | 32,8 | 6.40 | 7081 |
| (50) | 33,1 | 2.70 | 2986 |
| (51) | 33,5 | 2.33 | 2579 |
| (52) | 34,4 | 3.64 | 4020 |
| (53) | 34,6 | 3.59 | 3965 |
| (54) | 35,3 | 3.91 | 4319 |
| (55) | 35,4 | 3.88 | 4293 |
| (56) | 36,0 | 2.18 | 2406 |
| (57) | 36,7 | 3.06 | 3379 |
| (58) | 37,0 | 2.90 | 3203 |
| (59) | 37,6 | 3.59 | 3967 |
| (60) | 38,3 | 2.02 | 2229 |
| (61) | 38,9 | 3.17 | 3503 |
| (62) | 39,3 | 3.87 | 4282 |
| (63) | 40,1 | 3.33 | 3680 |
| (64) | 40,9 | 3.22 | 3560 |
| (65) | 41,6 | 3.12 | 3445 |
| (66) | 41,7 | 3.06 | 3378 |
| (67) | 41,9 | 2.65 | 2933 |
| (68) | 42,2 | 3.58 | 3961 |
| (69) | 42,6 | 2.51 | 2776 |
| (70) | 43,0 | 3.04 | 3362 |
| (71) | 44,5 | 1.94 | 2144 |
| (72) | 45,3 | 2.91 | 3217 |
| (73) | 45,8 | 2.02 | 2237 |
| (74) | 46,5 | 2.57 | 2841 |
| (75) | 47,0 | 1.84 | 2037 |
| (76) | 47,4 | 1.91 | 2107 |
| (77) | 48,3 | 2.42 | 2671 |
| (78) | 49,3 | 1.70 | 1883 |

### Beispiel 6 - Bestimmung der Reinheit der erfindungsgemäßen Dihydrochloridsalze

Die erfindungsgemäßen röntgenographischen Dihydrochloridsalze wurde mittels HPLC auf ihre Reinheit getestet. Als Vergleich wurden ebenfalls kommerziell erhältliche bzw. entsprechend dem Stand der Technik selbst hergestellte Dihydrochloridsalze untersucht.

Das Imipridon-201-dihydrochlorid gemäß Stand der Technik wurde nach dem in den Beispielen 1 und 2 von EP 2 968 294 B1 beschriebenen Verfahren hergestellt.

Das Imipridon-206-dihydrochlorid gemäß Stand der Technik wurde nach dem in den Beispielen 1 und 2 von EP 2 968 294 B1 beschriebenen Verfahren hergestellt mit dem Unterschied, dass anstelle von N-(2-Methylbenzyl)-4,5-dihydro-1H-imidazol-2-amin (Verbindung (2)) N-(2,4-Difluorbenzyl)-4,5-dihydro-1H-imidazol-2-amin (Verbindung (3) verwendet wurde.

Die HPLC-Messungen wurden mit einer RP C18-Säule 5µm (Korngröße des Säulenmaterials) mit den Dimensionen 250x4,6mm und einem DAD-Detektor durchgeführt. Hier noch ein Auszug aus unserer SOP:
Die Bestimmung der Identität, Reinheit und Gehalt von ONC201HCl erfolgt durch RP-HPLC. Die Chromatographie erfolgt durch eine apolare stationäre Phase (C18) mit einer mobilen Phase unter hohem Druck. Die Elution von ONC201HCl und mögliche Verunreinigungen wird durch die Absorptionsfähigkeit von der stationären C18-Phase und der Elutionskraft der mobilen Phase erreicht.

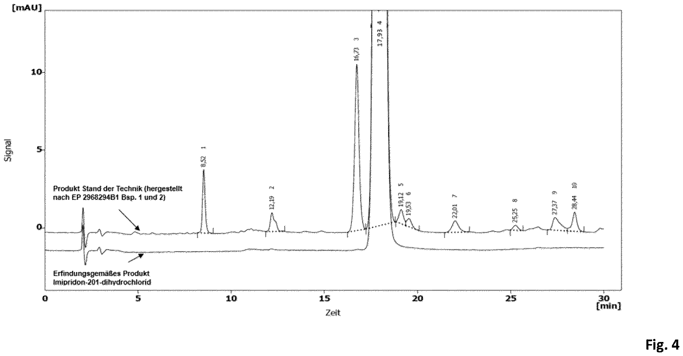

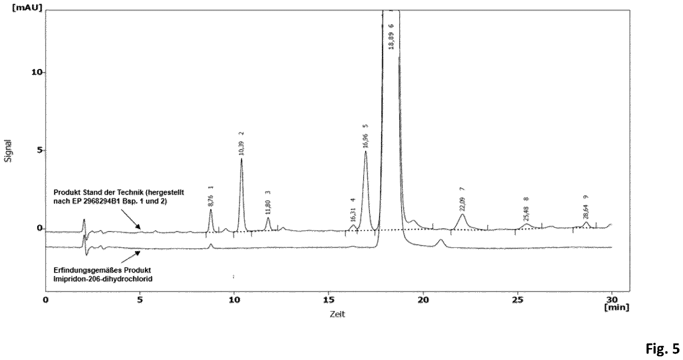

Die Ergebnisse sind in der **Figur 4** für Imipridon-201-dihydrochlorid und in **Figur 5** für Imipridon-206 dihydrochlorid dargestellt. Jeweils sowohl für die erfindungsgemäße röntgenographische Variante und die Vergleichsprobe.

Wie in **Figur 4** **und** **5** gezeigt, weisen die Dihydrochloridsalze von Imipridon-201 und Imipridon-206 chemisch eine deutlich verbesserte Reinheit gegenüber Dihydrochloridsalzen aus dem Stand der Technik.

Die Fläche der Peaks der Chromatogramme werden dabei als Maß für die Konzentration der Verunreinigung genommen. Die Nachweisgrenze liegt dabei bei 0,05 % Peakfläche bezogen auf die Gesamtpeakfläche, da kleinere Peaks auch auf eine Messungenauigkeit zurückzuführen sein können.

Die HPLC- Untersuchungen zeigen im erfindungsgemäßen Chromatogramm von Imipridon-201-dihydrochlorid 0 % Verunreinigungen oberhalb der Nachweisgrenze. Die Reinheit liegt somit bei 100 % (siehe auch Tabelle 3).

**Tabelle 3: Flächen der Peaks des Chromatogramms des erfindungsgemäßen Dihydrochlorid-Salzes von Imipridon-201 aus Figur 4**

| **Stoff** | **Flächenanteil [%]** |
|---|---|
| Imipridon-201-HCl | 100,00 |

Dagegen sind im Chromatogramm von Imipridon-201-dihydrochlorid gemäß Stand der Technik sieben Peaks mit einem Flächenanteil von über 0,1 % zu finden (siehe Tabelle 4). Einer der Peaks mit einer Retentionszeit von 16,73 macht sogar etwa 1,5 % der Gesamtkonzentration der Probe aus

**Tabelle 4: Flächen der Peaks des Chromatogramms des Dihydrochlorid-Salzes von Imipridon-201 gemäß Stand der Technik aus Figur 4. Für die Verunreinigungen sind die Retentionszeiten in min) in Klammern angegeben.**

| **Stoff** | **Flächenanteil [%]** |
|---|---|
| lmipridon-201-HCl | 97,146 |
| Verunreinigung 1 (8,52) | 0,346 |
| Verunreinigung 2 (12,19) | 0,181 |
| Verunreinigung 3 (16,73) | 1,517 |
| Verunreinigung 4 (19,12) | 0,131 |
| Verunreinigung 5 (22,01) | 0,157 |
| Verunreinigung 6 (27,37) | 0,2 |
| Verunreinigung 7 (28,44) | 0,19 |

Die HPLC-Untersuchungen zeigen im erfindungsgemäßen Chromatogramm von Imipridon-206-dihydrochlorid lediglich zwei Verunreinigungen oberhalb der Nachweisgrenze. Beiden liegen jeweils unterhalb von 0,1 %. Die Reinheit beträgt 99,9 % (siehe auch Tabelle 5).

**Tabelle 5: Flächen der Peaks des Chromatogramms des erfindungsgemäßen Dihydrochlorid-Salzes von Imipridon-206 aus Figur 5**

| **Stoff** | **Flächenanteil [%]** |
|---|---|
| Imipridon-201-HCl | 99,89 |
| Verunreinigung 1 (8,76) | 0,024 |
| Verunreinigung 2 (20,93) | 0,085 |

Dagegen sind im Chromatogramm von Imipridon-206-dihydrochlorid gemäß Stand der Technik sieben Peaks mit einem Flächenanteil von über 0,05 % zu finden (siehe Tabelle 6). Einer der Peaks mit einer Retentionszeit von 16,73 macht sogar etwa 0,78 % der Gesamtkonzentration der Probe aus.

**Tabelle 6: Flächen der Peaks des Chromatogramms des Dihydrochlorid-Salzes von Imipridon-206 gemäß Stand der Technik aus Figur 5. Für die Verunreinigungen sind die Retentionszeiten in min) in Klammern angegeben.**

| **Stoff** | **Flächenanteil [%]** |
|---|---|
| Imipridon-206-HCl | 98,042 |
| Verunreinigung 1 (8,76) | 0,130 |
| Verunreinigung 2 (10,39) | 0,462 |
| Verunreinigung 3 (11,8) | 0,099 |
| Verunreinigung 4 (16,31) | 0,055 |
| Verunreinigung 5 (16,96) | 0,786 |
| Verunreinigung 6 (22,09) | 0,269 |
| Verunreinigung 7 (25,48) | 0,19 |

### Beispiel 7 - Langzeitstabilität der erfindungsgemäßen Dihydrochloridsalze

Zur Bestimmung der ICH-konformen Langzeitstabilität wurden die erfindungsgemäßen Dihydrochloridsalze von Imipridon-201 und Imipridon-206 wie folgt gelagert. ICH-konforme Langzeitstabilitätsstudien schreiben umfangreiche Untersuchungen an drei Chargen bei unterschiedlichen Bedingungen fest. So sind für die Klimazonen I und II Stabilitätsstudien bei 25°C ± 2°C und 60% r.F. ± 5% r.F. (relative Feuchte) vorgeschrieben. Hinzu kommen je nach Lagerungsbedingungen des Wirkstoffes die entsprechenden Temperaturen wie z.B. 5°C ± 3°C oder -20°C ± 5°C. Sog. "Accelerated Studies" dienen dazu, unter Bedingungen wie z.B. 40°C ± 2°C und 75% r.F. ± 5% r.F. frühzeitig mögliche Abbauprodukte während der Lagerung zu erkennen und ggf. Extrapolationen vorzunehmen. Nach festgelegten Zeitpunkten, i.d.R. 0, 3, 6, 9, 12, 18, 24 und 36 Monate, werden Proben entnommen und die Reinheit analytisch mittels HPLC geprüft.

Einzelverunreinigungen zum Zeitpunkt t=0 von mehr als 0,1 % scheiden von vornherein für ICH-konforme Langzeitstabilitätsuntersuchungen aus. Aus diesem Grund sind vergleichende Untersuchungen nicht möglich. Es ist zu erwarten, dass ein derartiges Verunreinigungsprofil im Laufe der Lagerung zu vermehrten Abbauprodukten führen kann, die zusätzlich aus den bereits in höheren Konzentrationen vorhandenen Verunreinigungen entstehen.

### Ergebnis der Langzeitstudie

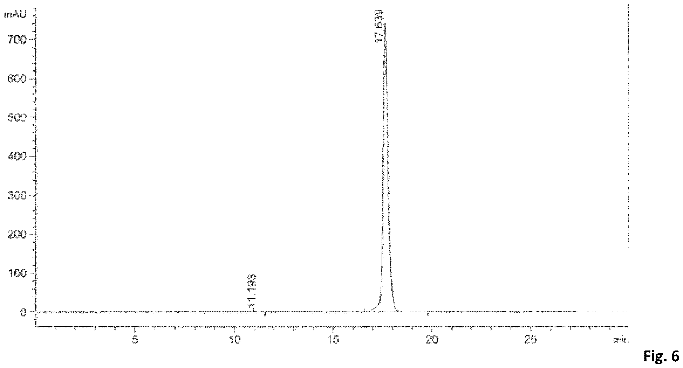

Es konnten für Imipridon-201-dihydrochlorid über 36 Monate und für Imipridon-206-dihydrochlorid über 24 Monate keinerlei Veränderungen der chemischen Zusammensetzung festgestellt werden (Gehalt HPLC %area). Bei der Auswertung der Ergebnisse konnten überraschenderweise lediglich bei einzelnen Chargen Verunreinigungen in Mengen von max. 0,10% bzw. bis zum "Reporting Threshold" von 0,05% beispielsweise in Höhe von 0,03% detektiert werden. Diese Ergebnisse liegen im Bereich von analytischen Schwankungen. Beispielhaft wird in **Figur 6** ein Chromatogramm mit Ergebnistabelle für eine Charge Imipridon-201-dihydrochlorid gezeigt, die über 36 Monate bei 25°C und 60% r.F. gelagert wurde.

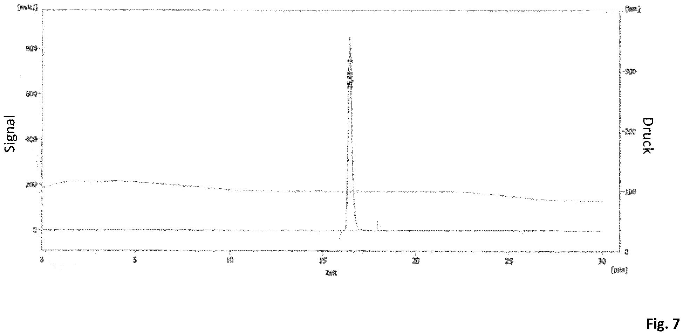

**Figur 7** zeigt beispielhaft das Chromatogramm einer Charge von Imipridon-206-dihydrochlorid, die über 24 Monate bei 25°C und 60% r.F. gelagert wurde.

**Tabelle 7: Imipridon-201-HCl Langzeitstabilität**

| **Bestandteil** | 0 | 36 Monate |
|---|---|---|
| Imipridon-201-HCl | 100% | 100% |
| Verunreinigungen | 0% | 0% |

**Tabelle 8: Imipridon-206-HCl Langzeitstabilität**

| **Bestandteil** | 0 | 24 Monate |
|---|---|---|
| Imipridon-206-HCl | 100% | 100% |
| Verunreinigungen | 0% | 0% |

### Beispiel 8 - "Accelerated Studies" zur Langzeitstabilität

Für die erfindungsgemäßen röntgenographischen Kristallformen liegen Ergebnisse aus "Accelerated Studies" gemäß ICH-konformen Langzeitstabilitätsstudien unter den o.g. Bedingungen vor.

**Tabelle 9: Imipridon-201-HCl "Accelerated Studies"**

| **Bestandteil** | 0 | 6 Monate |
|---|---|---|
| Imipridon-201-HCl | 100% | 100% |
| Verunreinigung 1 | 0% | 0% |
| Verunreinigung 2 | 0% | 0% |
| Verunreinigung 3 | 0% | 0% |

**Tabelle 10: Imiridon-201-HCl "Accelerated Studies"**

| **Bestandteil** | 0 Monate | 6 Monate |
|---|---|---|
| Imipridon-206-HCl | 100% | 100% |
| Verunreinigung 1 | 0% | 0% |
| Verunreinigung 2 | 0% | 0% |
| Verunreinigung 3 | 0% | 0% |

Selbst die "Accelerated Studies" bei Lagerung unter Stressbedingungen wie erhöhter Temperatur und relativer Luftfeuchtigkeit (40°C und 75% Luftfeuchte) über 6 Monate zeigten keine Veränderung der Reinheit.

Weiter wurde unter ICH-Bedingungen die Langzeitstabilität von pharmazeutischen Zusammensetzungen (Hartgelatinekapseln) mit den erfindungsgemäßen röntgenographischen Kristallformen in Mischung mit pharmazeutisch üblichen Hilfsstoffen überprüft. Auch hier zeigen sich über 24 Monate keine Veränderungen in der chemischen Stabilität.

Hinsichtlich weiterer vorteilhafter Ausgestaltungen der erfindungsgemäßen Vorrichtung wird zur Vermeidung von Wiederholungen auf den allgemeinen Teil der Beschreibung sowie auf die beigefügten Ansprüche verwiesen.

Schließlich sei ausdrücklich darauf hingewiesen, dass die voranstehend beschriebenen Ausführungsbeispiele der erfindungsgemäßen Vorrichtung lediglich zur Erörterung der beanspruchten Lehre dienen, diese jedoch nicht auf die Ausführungsbeispiele einschränken.

## Patentansprüche

1. Dihydrochloridsalz von Imipridon-201 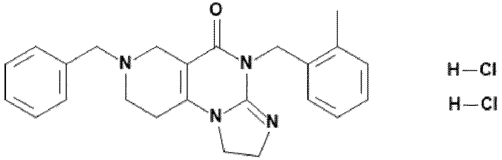 in kristalliner Form, **dadurch gekennzeichnet, dass** das Röntgen-Pulverdiffraktogramm dieses Salzes bei Verwendung von Cu K-α-Strahlung bei 25°C mindestens 3 der folgenden 2Θ (2 Theta)-Werte mit einer Fehlertoleranz von jeweils ± 0.2 aufweist:
| | 2Θ (2Theta)-Werte in ° |
|---|---|
| (2) | 6,9 |
| (3) | 7,7 |
| (5) | 9,6 |
| (8) | 12,9 |
| (13) | 15,5 |
| (29) | 21,2 |
| (30) | 21,4 |
| (32) | 22,5 |
| (33) | 23,1 |
| (38) | 25,3 |
| (41) | 26,8 |

2. Dihydrochloridsalz von Imipridon-201 nach Anspruch 1, **dadurch gekennzeichnet, dass** das Röntgen-Pulverdiffraktogramm dieses Salzes bei Verwendung von Cu K-α-Strahlung bei 25°C mindestens 4, vorzugsweise mindestens 5, besonders bevorzugt sämtliche der in Anspruch 1 genannten 2Θ (2 Theta)-Werte mit einer Fehlertoleranz von jeweils ± 0.2 aufweist.

3. Dihydrochloridsalz von Imipridon-201 nach Anspruch 1 oder 2, **dadurch gekennzeichnet, dass** das Röntgen-Pulverdiffraktogramm dieses Salzes zusätzlich mindestens zwei, mindestens vier, mindestens sechs, mindestens acht, mindestens zehn, mindestens zwölf oder sämtliche der folgenden 2Θ (2 Theta)-Werte mit einer Fehlertoleranz von jeweils ± 0.2 aufweist: 4,8, 8,2 11,3, 12,0, 13,9, 14,3, 14,7, 15,1, 16,0, 16,2, 16,4, 16,7, 17,1, 17,7, 18,1, 18,3, 18,5, 18,7, 19,0, 19,4, 19,7, 20,3, 20,7, 22,0, 23,6, 24,1, 24,4, 24,9, 25,7, 25,9, 27,7, 28,0, 28,8, 29,2, 29,7, 31,0, 31,3, 31,9, 32,5, 33,7, 34,4, 34,7, 35,5, 36,5, 37,9, 38,3, 38,6, 39,3, 39,9.

4. Dihydrochloridsalz von Imipridon-201 nach einem der Ansprüche 1 bis 3, **dadurch gekennzeichnet, dass** das Salz gebundenes Wasser enthält und im Infrarot-Spektrum OH-Streckschwingungen von Wasser im Bereich von 3000 cm⁻¹ bis 3700 cm⁻¹ aufweist.

5. Dihydrochlorid-Salz von Imipridon-201 nach einem der Ansprüche 1 bis 4, **dadurch gekennzeichnet, dass** das Salz die ICH-konforme Reinheit von 99,0 bis 100% aufweist.

6. Dihydrochloridsalz von Imipridon-206 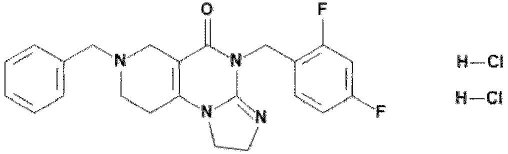 in kristalliner Form, **dadurch gekennzeichnet, dass** das Röntgen-Pulverdiffraktogramm dieses Salzes bei Verwendung von Cu K-α-Strahlung bei 25°C mindestens 3 der folgenden 2Θ (2 Theta)-Werte mit einer Fehlertoleranz von jeweils ± 0.2 aufweist:
| | 2Θ (2Theta)-Werte in ° |
|---|---|
| (12) | 14,2 |
| (25) | 21,3 |
| (33) | 25,5 |
| (34) | 26,0 |
| (38) | 27,7 |

7. Dihydrochloridsalz von Imipridon-206 nach Anspruch 6, **dadurch gekennzeichnet, dass** das Röntgen-Pulverdiffraktogramm dieses Salzes bei Verwendung von Cu K-α-Strahlung bei 25°C mindestens 4, vorzugsweise mindestens 5, besonders bevorzugt sämtliche der in Anspruch 1 genannten 2Θ (2 Theta)-Werte aufweist.

8. Dihydrochloridsalz von Imipridon-206 nach Anspruch 6 oder 7, **dadurch gekennzeichnet, dass** das Röntgen-Pulverdiffraktogramm dieses Salzes zusätzlich mindestens zwei, vier, sechs, acht, zehn, zwölf oder mehr der folgenden 2Θ (2 Theta)-Werte aufweist: 6,0, 6,5, 6,9, 7,7, 9,0, 10,0, 11,5, 12,0, 13,0, 13,5, 13,6, 14,8, 15,6, 16,1, 16,6, 16,9, 18,1, 18,9, 19,0, 19,6, 19,9, 20,1, 20,4, 21,8, 22,1, 22,5, 22,9, 23,7, 23,8, 24,9, 26,3, 27,1, 27,4, 28,2, 28,7, 29,1, 29,6, 30,2, 30,4, 30,7, 31,0, 31,4, 32,1, 32,8, 33,1, 33,5, 34,4, 34,6, 35,3, 35,4, 36,0, 36,7, 37,0, 37,6, 38,3, 38,9, 39,3, 40,1, 40,9, 41,6, 41,7, 41,9, 42,2, 42,6, 43,0, 44,5, 45,3, 45,8, 46,5, 47,0, 47,4, 48,3, 49,3.

9. Dihydrochlorid-Salz von Imipridon-201 nach einem der Ansprüche 1 bis 5 oder Dihydrochlorid-Salz von Imipridon-206 nach einem der Ansprüche 6 bis 8, **dadurch gekennzeichnet, dass** das Dihydrochlorid-Salz eine ICH-konforme Langzeitstabilität bei 25°C und 60 % r.F. von mindestens 12 Monaten aufweist, bevorzugt von mindestens 24 Monaten, besonders bevorzugt von mindestens 36 Monaten.

10. Verfahren zur Herstellung eines Dihydrochloridsalzes von Imipridon-201, wobei Imipridon-201 in einer sauren wässrigen Lösung gelöst und anschließend kristallisiert wird, **dadurch gekennzeichnet, dass** die Kristallisation durch Lösungsmittelaustausch ausgelöst wird, wobei die saure wässrige Lösung HCl enthält, und wobei das Austauschlösungsmittel, vorzugsweise ausgewählt ist aus Ketonen, Estern, Alkoholen oder Ethern, wobei das Keton bevorzugt Aceton ist.

11. Verfahren nach Anspruch 10, umfassend die folgenden Schritte:
a) Erwärmen einer HCl-haltigen wässrigen Lösung; wobei bevorzugt 0,5 bis 5 ml HCl-haltiger wässriger Lösung pro 1 g Imipridon-201, bevorzugter 1 bis 3 ml HCl-haltiger wässriger Lösung pro 1 g Imipridon-201 erwärmt wird, und wobei die HCl-haltigen wässrigen Lösung bevorzugt auf eine Temperatur im Bereich von 50-70°C erwärmt wird;
b) Zugabe von Imipridon-201;
c) Einstellung des pH der Mischung auf einen Wert im Bereich von 3 bis 5, bevorzugt auf einen Bereich von 3,5 bis 4,5 besonders bevorzugt durch Zutropfen weiterer HCl-haltiger wässriger Lösung;
d) gegebenenfalls Filtration der Lösung, insbesondere mit einer Porengröße von höchstens 1 µm;
e) Einstellen der Temperatur auf einen Wert im Bereich von 30 bis 70 °C, bevorzugt im Bereich von 40 bis 60°C;
f) unter Rühren tropfenweise Zugabe von 10%iger HCl bis zu einem pH-Wert im Bereich von 0,4 bis 2,0, bevorzugt im Bereich von 0,6 bis 1,5, besonders bevorzugt im Bereich von 0,8 bis 1,0;
g) Zugabe des Austauschlösungsmittels, bevorzugt mit 5 bis 15 ml, besonders bevorzugt 8 bis 12 ml Austauschlösungsmittels pro 1 g Imipridon-201;
h) Abkühlen auf eine Temperatur im Bereich von 10 bis 30 °C, bevorzugt im Bereich von 15 bis 25 °C, besonders bevorzugt ungefähr Raumtemperatur;
i) Zugabe einer weiteren Menge des Austauschlösungsmittels bevorzugt mit 30 bis 50 ml Lösungsmittel pro 1 g Imipridon-201, wobei die Zugabe bevorzugt über einen Zeitraum von mindestens 2 h, besonders bevorzugt über einen Zeitraum von mindestens 4 h erfolgt;
j) Rühren der Lösung mindestens 0,5 h, bevorzugt mindestens 1,0 h bei Raumtemperatur;
k) Abtrennung des Präzipitats, bevorzugt durch Filtration, besonders bevorzugt mit einer Porengröße von höchstens 1 µm;
l) Waschen des Präzipitats mit 1,0 bis 10,0 ml, bevorzugt 2,0 bis 7,0 ml, besonders bevorzugt 3,0 bis 5,0 ml des Austauschlösungsmittels; und
m) Trocknung des Präzipitats, wobei die Trocknung über einen Zeitraum von mindestens 10 h, bevorzugt mindestens 14 h, besonders bevorzugt mindestens 16 h stattfindet, wobei die Temperatur während der Trocknung bevorzugt im Bereich von 30 bis 70 °C, besonders bevorzugt im Bereich von 40 bis 60 °C, insbesondere im Bereich von 45 bis 55 °C liegt und der Druck im Bereich von 10 bis 100 mbar, bevorzugt im Bereich von 20 bis 40 mbar.

12. Verfahren nach Anspruch 11, wobei die HCl-haltige wässrige Lösung in Schritt a) eine 10 % HCl-Lösung im Bereich von 10 bis 40 Vol.%, bevorzugt im Bereich von 15 bis 30 Vol.%, besonders bevorzugt im Bereich von 20 bis 25 Vol.% enthält.

13. Verfahren nach einem der Ansprüche 11 oder 12, ferner umfassend die Schritte:
n) Zugabe des Präzipitats zu einem Alkohol, ausgewählt aus der Gruppe der Alkohole mit 1 bis 4 C-Atomen, bevorzugt Ethanol, besonders bevorzugt 96 %iger Ethanol (EtOH 96 %), wobei bevorzugt 2 bis 40 ml, besonders bevorzugt 10 bis 30 ml, vorzugsweise 15 - 20 ml des Alkohols pro 1 g des Präzipitats verwendet wird;
o) Erhitzen der Mischung unter Rühren mit einer Wärmequelle mit einer Temperatur im Bereich von 50 bis 95 °C, bevorzugt 60 bis 90°C, besonders bevorzugt 70 bis 80 °C, wobei vorzugsweise verdampfendes Ethanol rückfließt,
p) Abkühlen auf eine Temperatur im Bereich von 20 bis 40 °C, bevorzugt 25 bis 35 °C über einen Zeitraum von mindestens 1 h, bevorzugt mindestens 1,5 h besonders bevorzugt mindestens 2,5 h;
q) Abtrennung des Präzipitats, bevorzugt durch Filtration, besonders bevorzugt mit einer Porengröße von höchstens 1 µm;
r) Optional Waschen des Präzipitats mit 0,1 bis 5 ml, bevorzugt 1 bis 3 ml, besonders bevorzugt 1,5 bis 2,5 ml des Alkohols pro 1 g des Präzipitats;
s) Waschen des Präzipitats mit 1,0 bis 8,0 ml, bevorzugt 2,0 bis 6,0 ml, besonders bevorzugt 3,5 bis 4,5 ml eines organischen Lösungsmittels, ausgewählt aus Ketonen, Estern, Alkoholen oder Ethern, wobei das Keton bevorzugt Aceton ist;
t) Trocknung des Feststoffs, wobei die Trocknung einen oder mehrere Trocknungsschritte aufweist und wobei bevorzugt mindestens ein Trocknungsschritt Vakuum-Trocknung über einen Zeitraum von 1 bis 5 Tagen, besonders bevorzugt über einen Zeitraum von 1,5 bis 4 Tagen ist, wobei die Temperatur während der Vakuum-Trocknung bevorzugt im Bereich von 50 bis 100 °C, besonders bevorzugt im Bereich von 65 bis 95 °C, insbesondere im Bereich von 75 bis 85 °C liegt.

14. Verfahren zur Herstellung von Imipridon-201, **gekennzeichnet durch** die folgenden Schritte:
a) Zugabe von N-(2-Methylbenzyl)-4,5-dihydro-1H-imidazol-2-amin (Verbindung (2)) zu einem alkoholischen Lösungsmittel, bevorzugt in einer Konzentration von 10 Gew-% bis 35 Gew-%, besonders bevorzugt von 15 Gew-% bis 30 Gew-%, insbesondere 20 bis 25 Gew-% Verbindung (2) bezogen auf das alkoholische Lösungsmittel, wobei das alkoholische Lösungsmittel bevorzugt Methanol ist;
b) Zugabe von 1-Benzyl-4-oxopiperidin-3-carbonsäuremethylester hydrochlorid (Verbindung (1)) in einer Konzentration von 15 bis 45 Gew-%, besonders bevorzugt von 20 bis 40 Gew-%, insbesondere 25 bis 35 Gew-% Verbindung (2) bezogen auf das alkoholische Lösungsmittel;
c) Zugabe von Natriummethanolat (NaOMe) in einer Konzentration von 5 Vol.-% bis 35 Vol.-%, besonders bevorzugt von 10 Vol.-% bis 30 Vol.-%, insbesondere 15 Vol.-% bis 25 Vol.-% NaOMe bezogen auf das alkoholische Lösungsmittel; wobei das NaOMe bevorzugt zugetropft wird und wobei die Temperatur der Lösung bevorzugt im Bereich von 20 bis 50 °C, besonders bevorzugt im Bereich von 30 bis 40 °C, insbesondere im Bereich von 33 bis 37 °C;
d) Einstellen des pH auf einen Wert von mehr als 10, bevorzugt mehr als 11, bevorzugt mehr als 12;
e) Rühren der Reaktionsmischung über einen Zeitraum von mehr als 5 h, bevorzugt im Bereich von 10 bis 30 h, besonders bevorzugt im Bereich von 15 bis 25 h;
f) Einstellen des pH auf einen Wert im Bereich von 8,0 bis 10,0, bevorzugt im Bereich von 8,5 bis 9,5, besonders bevorzugt etwa 9,0; wobei der pH-Wert bevorzugt **durch** Zugabe von 10 %-iger HCl eingestellt wird, wobei die Zugabe bevorzugt über einen Zeitraum von 60 bis 180 min durchgeführt wird, besonders bevorzugt über einen Zeitraum von 100 bis 140 min;
g) Rühren der Reaktionsmischung über einen Zeitraum im Bereich von 6 bis 20 h, bevorzugt über einen Zeitraum von im Bereich von 10 bis 14 h;
h) Abtrennen des Reaktionsprodukts, Imipridon-201 vom Lösungsmittel, insbesondere **durch** Filtration;
i) optional Reinigen des Reaktionsprodukts, wobei das Reinigen einen oder mehrere Reinigungsschritte aufweist, und wobei bevorzugt mindestens ein Reinigungsschritt die Zugabe eines Alkohols, insbesondere Methanol und die anschließende Abtrennung des Alkohols, insbesondere **durch** Filtration umfasst;
j) Trocknung des Reaktionsprodukts, wobei die Trocknung über einen Zeitraum von 1 bis 4 Tagen, besonders bevorzugt über einen Zeitraum von 1,5 bis 4 Tagen durchgeführt wird, wobei die Temperatur während der Vakuum-Trocknung bevorzugt im Bereich von 30 bis 70 °C, besonders bevorzugt im Bereich von 40 bis 60 °C, insbesondere im Bereich von 45 bis 55 °C liegt.

15. Verfahren nach Anspruch 14, ferner enthaltend die folgenden Schritte:
k) Zugabe des Reaktionsprodukts Imipridon-201 aus Schritt j) zu einem Alkohol, ausgewählt aus Methanol, Ethanol, Methanol, Propanol, Isopropanol, n-Butanol, bevorzugt Methanol (MetOH), wobei bevorzugt 0,5 bis 10 ml, besonders bevorzugt 1,0 bis 6 ml, vorzugsweise 1,5 - 2,5 ml des Alkohols pro 1 g des Präzipitats verwendet wird;
l) Erhitzen der Mischung unter Rühren auf eine Temperatur im Bereich von 40 bis 80 °C, bevorzugt 50 bis 70°C, besonders bevorzugt 60 bis 65 °C, und Zugabe von weiterem Alkohol, vorzugsweise MetOH bis Imipridon-201 gelöst ist;
m) Abtrennung von Resten, bevorzugt durch Filtration besonders bevorzugt in einen Kolben mit Rührwerk und Rückflusskühler;
n) Erhitzen der Lösung unter Rühren zum Siedepunkt;
o) Ausfällen von Imipridon-201 durch Abkühlen, wobei das Abkühlen einen oder mehrere Abkühlungsschritte aufweist und wobei bevorzugt mindestens ein Abkühlungsschritt über einen Zeitraum von 10 bis 24 h stattfindet, besonders bevorzugt über einen Zeitraum von 12 bis 20 h;
p) Waschen des Präzipitats mit 0,1 bis 3,0 ml, bevorzugt 0,2 bis 1,0 ml, besonders bevorzugt 0,5 bis 0,7 ml eines Alkohols;
q) Trocknung des Präzipitats, wobei die Trocknung über einen Zeitraum von mindestens 10 h, bevorzugt mindestens 14 h, besonders bevorzugt mindestens 16 h stattfindet, wobei die Temperatur während der Trocknung bevorzugt im Bereich von 30 bis 70 °C, besonders bevorzugt im Bereich von 40 bis 60 °C, insbesondere im Bereich von 45 bis 55 °C liegt und der Druck im Bereich von 10 bis 100 mbar, bevorzugt im Bereich von 20 bis 40 mbar.

16. Verfahren zur Herstellung eines Dihydrochloridsalzes von Imipridon-206, wobei Imipridon-206 in einer sauren wässrigen Lösung gelöst und anschließend kristallisiert wird, **dadurch gekennzeichnet, dass** die Kristallisation durch Lösungsmittelaustausch ausgelöst wird, wobei die saure wässrige Lösung HCl enthält, und wobei das Lösungsmittel vorzugsweise ausgewählt ist aus Ketonen, Estern, Alkoholen oder Ethern, wobei das Keton bevorzugt Aceton ist.

17. Verfahren nach Anspruch 16, umfassend die folgenden Schritte:
a) Erwärmen einer HCl-haltigen wässrigen Lösung; wobei die bevorzugt mit 0,5 bis 5 ml HCl-haltiger wässriger Lösung pro 1 g Imipridon-206, bevorzugter mit 1 bis 3 ml HCl-haltiger wässriger Lösung pro 1 g Imipridon-206, wobei die HCl-haltigen wässrigen Lösung bevorzugt auf eine Temperatur im Bereich von 55 bis 65 °C erhitzt wird;
b) Zugabe von Imipridon-206;
c) Einstellen des pH der Mischung auf einen Wert im Bereich von 3 bis 5 bevorzugt auf einen Bereich von 3,5 bis 4,5; besonders bevorzugt durch Zutropfen weiterer HCl-haltiger wässriger Lösung;
d) gegebenenfalls Feinfiltration der Lösung;
e) Einstellen der Temperatur auf einen Wert im Bereich von 30 bis 70 °C, bevorzugt im Bereich von 40 bis 60 °C,
f) unter Rühren tropfenweise Zugabe von 10%iger HCl bis zu einem pH Wert im Bereich von 0,5 bis 3,0, bevorzugt im Bereich von 1,0 bis 1,8, besonders bevorzugt im Bereich von 1,3 bis 1,5;
g) Zugabe des organischen Lösungsmittels, bevorzugt mit 5 bis 15 ml, besonders bevorzugt 8 bis 12 ml Lösungsmittel pro 1 g Imipridon-206;
h) Abkühlen auf Raumtemperatur; und
i) Zugabe einer weiteren Menge des organischen Lösungsmittels bevorzugt mit 30 bis 50 ml Lösungsmittel pro 1 g Imipridon-206, wobei die Zugabe bevorzugt über einen Zeitraum von mindestens 2 h, besonders bevorzugt über einen Zeitraum von mindestens 4 h;
j) Rühren der Lösung mindestens 0,5 h, bevorzugt mindestens 1,0 h bei Raumtemperatur;
k) Abtrennung des Präzipitats, bevorzugt durch Filtration, besonders bevorzugt mit einer Porengröße von höchstens 1 µm;
l) Waschen des Präzipitats mit 1,0 bis 10,0 ml, bevorzugt 2,0 bis 7,0 ml, besonders bevorzugt 3,0 bis 5,0 ml des Austauschlösungsmittels; und
m) Trocknung des Präzipitats, wobei die Trocknung über einen Zeitraum von mindestens 10 h, bevorzugt mindestens 14 h, besonders bevorzugt mindestens 16 h stattfindet, wobei die Temperatur während der Trocknung bevorzugt im Bereich von 30 bis 70 °C, besonders bevorzugt im Bereich von 40 bis 60 °C, insbesondere im Bereich von 45 bis 55 °C liegt und der Druck im Bereich von 10 bis 100 mbar, bevorzugt im Bereich von 20 bis 40 mbar.

18. Verfahren nach Anspruch 17, wobei die wobei die saure wässrige Lösung in Schritt a) eine 10 % HCl-Lösung im Bereich von 10 bis 40 Vol.%, bevorzugt im Bereich von 15 bis 30 Vol.%, besonders bevorzugt im Bereich von 20 bis 25 Vol.% enthält.

19. Verfahren nach einem der Ansprüche 17 oder 18, ferner umfassend die Schritte:
n) Zugabe des Präzipitats zu einem Alkohol, ausgewählt aus der Gruppe der Alkohole mit 1 bis 4 C-Atomen, bevorzugt Ethanol, besonders bevorzugt 96 %iger Ethanol (EtOH 96 %), wobei bevorzugt 1 bis 25 ml, besonders bevorzugt 2 bis 15 ml, vorzugsweise 4 bis 8 ml des Alkohols pro 1 g des Präzipitats verwendet wird;
o) optional weitere Zugabe von 0,1 bis 10 ml, bevorzugt 1 bis 5 ml, besonders bevorzugt 1 bis 3 ml des Alkohols pro 1 g des Präzipitats;
p) Erhitzen der Mischung unter Rühren mit einer Wärmequelle mit einer Temperatur im Bereich von 50 bis 95 °C, bevorzugt 60 bis 90°C, besonders bevorzugt 70 bis 80 °C, wobei vorzugsweise verdampfendes Ethanol rückfließt,
q) Abkühlen auf eine Temperatur im Bereich von 20 bis 40 °C, bevorzugt 25 bis 35 °C über einen Zeitraum von mindestens 1 h, bevorzugt mindestens 1,5 h besonders bevorzugt mindestens 2,5 h;
r) Abtrennung des Präzipitats, insbesondere durch Filtration;
s) Optional Waschen des Präzipitats mit 0,1 bis 5 ml, bevorzugt 1 bis 3 ml, besonders bevorzugt 1,5 bis 2,5 ml des Alkohols pro 1 g des Präzipitats;
t) Waschen des Präzipitats mit 1,0 bis 8,0 ml, bevorzugt 2,0 bis 6,0 ml, besonders bevorzugt 3,5 bis 4,5 ml eines organischen Lösungsmittels, ausgewählt aus Ketonen, Estern, Alkoholen oder Ethern, wobei das Keton bevorzugt Aceton ist; und
u) Trocknung des Feststoffs, wobei die Trocknung einen oder mehrere Trocknungsschritte aufweist und wobei bevorzugt mindestens ein Trocknungsschritt Vakuum-Trocknung über einen Zeitraum von 1 bis 5 Tagen, besonders bevorzugt über einen Zeitraum von 1,5 bis 4 Tagen ist, wobei die Temperatur während der Vakuum-Trocknung bevorzugt im Bereich von 50 bis 100 °C, besonders bevorzugt im Bereich von 65 bis 95 °C, insbesondere im Bereich von 75 bis 85 °C liegt.

20. Verfahren zur Herstellung von Imipridon-206, **gekennzeichnet durch** die folgenden Schritte:
a) Zugabe von N-(2-Methylbenzyl,4-Difluorbenzyl)-4,5-dihydro-1H-imidazol-2-amin (Verbindung (3) zu einem alkoholischen Lösungsmittel, bevorzugt in einer Konzentration von 15 bis 35 Gew.-%, besonders bevorzugt von 20 bis 30% Gew.-%, insbesondere 22 bis 28 Gew.-% Verbindung (3) bezogen auf das alkoholische Lösungsmittel, wobei das alkoholische Lösungsmittel bevorzugt Methanol ist;
b) Zugabe von 1-Benzyl-4-oxopiperidin-3-carbonsäuremethylester hydrochlorid (Verbindung (1)) in einer Konzentration von 20 bis 40 Gew.-%, besonders bevorzugt von 25 bis 35 Gew.-%, insbesondere 28 bis 33 Gew.-% Verbindung (1) bezogen auf das alkoholische Lösungsmittel;
c) Zugabe von Natriummethanolat (NaOMe) in einer Konzentration von 10 Vol-% bis 40 Vol-%, besonders bevorzugt von 20 Vol-% bis 30 Vol-%, insbesondere 23 Vol-% bis 27 Vol-% NaOMe bezogen auf das alkoholische Lösungsmittel; wobei das NaOMe bevorzugt zugetropft wird und wobei die Temperatur der Lösung bevorzugt im Bereich von 20 bis 50 °C liegt, besonders bevorzugt im Bereich von 30 bis 40 °C, insbesondere im Bereich von 33 bis 37 °C;
d) Einstellen des pH auf einen Wert von mehr als 10, bevorzugt mehr als 11, bevorzugt mehr als 12;
e) Rühren der Reaktionsmischung über einen Zeitraum von mehr als 5 h, bevorzugt im Bereich von 10 bis 30 h, besonders bevorzugt im Bereich von 15 bis 25 h;
f) Einstellen des pH auf einen Wert von im Bereich von 8,0 bis 10,0, bevorzugt im Bereich von 8,5 bis 9,5, besonders bevorzugt etwa 9,0 mehr als 12; wobei der pH-Wert bevorzugt **durch** Zugabe von 10 %-iger HCl eingestellt wird, wobei die Zugabe bevorzugt über einen Zeitraum von 60 bis 180 h durchgeführt wird, besonders bevorzugt über einen Zeitraum von 100 bis 140 min;
g) Rühren der Reaktionsmischung über einen Zeitraum im Bereich von 6 bis 20 h, bevorzugt über einen Zeitraum von im Bereich von 10 bis 14 h;
h) Abtrennen des Reaktionsprodukts, Imipridon-206, vom Lösungsmittel, insbesondere **durch** Filtration;
i) optional Reinigen des Reaktionsprodukts, wobei das Reinigen einen oder mehrere Reinigungsschritte aufweist, und wobei bevorzugt mindestens ein Reinigungsschritt die Zugabe eines Alkohols, insbesondere Methanol und die anschließende Abtrennung des Alkohols, insbesondere **durch** Filtration umfasst; und
j) Trocknung des Reaktionsprodukts, wobei die Trocknung über einen Zeitraum von 1 bis 4 Tagen, besonders bevorzugt über einen Zeitraum von 1,5 bis 4 Tagen ist, wobei die Temperatur während der Vakuum-Trocknung bevorzugt im Bereich von 30 bis 70 °C, besonders bevorzugt im Bereich von 40 bis 60 °C, insbesondere im Bereich von 45 bis 55 °C liegt.

21. Verfahren nach Anspruch 18, ferner enthaltend die folgenden Schritte:
r) Zugabe von Imipridon-206 aus Schritt j) zu einem Alkohol, ausgewählt aus der Gruppe der Alkohole mit 1-4 C-Atomen Methanol, Ethanol, Propanol, Isopropanol, Butanol, bevorzugt 96%-igem Ethanol (EtOH), wobei bevorzugt 0,5 bis 10 ml, besonders bevorzugt 1,0 bis 6 ml, vorzugsweise 1,5 - 2,5 ml des Alkohols pro 1 g des Präzipitats verwendet wird;
s) Erhitzen der Mischung unter Rühren auf eine Temperatur im Bereich von 40 bis 80 °C, bevorzugt 50 bis 70°C, besonders bevorzugt 60 bis 65 °C, und Zugabe von weiterem Alkohol, vorzugsweise EtOH bis Imipridon-206 gelöst ist;
t) Abtrennung von Resten, bevorzugt durch Filtration besonders bevorzugt in einen Kolben mit Rührwerk und Rückflusskühler;
u) Erhitzen der Lösung unter Rühren zum Siedepunkt;
v) Ausfällen von Imipridon-206 durch Abkühlen, wobei das Abkühlen einen oder mehrere Abkühlungsschritte aufweist und wobei bevorzugt mindestens ein Abkühlungsschritt über einen Zeitraum von 10 bis 24 h stattfindet, besonders bevorzugt über einen Zeitraum von 12 bis 20 h;
w) Waschen des Präzipitats mit 0,1 bis 3,0 ml, bevorzugt 0,2 bis 1,0 ml, besonders bevorzugt 0,5 bis 0,7 ml eines Alkohols; und
x) Trocknung des Präzipitats, wobei die Trocknung über einen Zeitraum von mindestens 10 h, bevorzugt mindestens 14 h, besonders bevorzugt mindestens 16 h stattfindet, wobei die Temperatur während der Trocknung bevorzugt im Bereich von 30 bis 70 °C, besonders bevorzugt im Bereich von 40 bis 60 °C, insbesondere im Bereich von 45 bis 55 °C liegt und der Druck im Bereich von 10 bis 100 mbar, bevorzugt im Bereich von 20 bis 40 mbar.

22. Verfahren nach einem der Ansprüche 9 bis 21, **dadurch gekennzeichnet, dass** das Verfahren ohne Dioxan durchgeführt wird, bevorzugt ohne Lösungsmittel, die toxisch, krebserregend und/oder umweltschädlich sind.

23. Dihydrochlorid-Salz von Imipridon-201 gemäß einem der Ansprüche 1 bis 5, zur Verwendung als Medikament.

24. Dihydrochlorid-Salz von Imipridon-206 gemäß einem der Ansprüche 6 bis 9, zur Verwendung als Medikament.

25. Pharmazeutische Zusammensetzung zur Verwendung in der Behandlung von Krebs, **dadurch gekennzeichnet, dass** sie ein Dihydrochlorid-Salz von Imipridon-201 gemäß einem der Ansprüche 1 bis 5 enthält und/oder ein Dihydrochlorid-Salz von Imipridon-206 gemäß einem der Ansprüche 6 bis 8 sowie einen pharmazeutisch akzeptablen Hilfsstoff und/oder einen pharmazeutisch akzeptablen Träger.
